# EUROPEAN PATENT APPLICATION

(11) **EP 4 834 423 A1**
(43) Date of publication of application: **04.11.2026**
(21) Application number: 24907773.6
(22) Date of filing: 05.11.2024
(51) Int. Cl.: A61N 5/06, A61F 7/00, A61N 5/00

(54) **NEAR-INFRARED BODY CARE DEVICE**

(30) Priority: 22.12.2023 KR 20230190234; 26.09.2024 KR 20240131162; 10.10.2024 KR 20240137993; 10.10.2024 KR 20240137994
(71) Applicant: Withsunbio Co., Ltd., Hwasung-si, Gyeonggi-do 18469 (KR)
(72) Inventor: LEE, IL Ho, Hwaseong-si Gyeonggi-do 18434 (KR)
(74) Representative: Dondi, Silvia
(86) International application number: PCT/KR2024/017324
(87) International publication number: WO 2025/135488

(57) **Abstract**

The present disclosure relates to a near-infrared body care device, comprising: a support case supported on a floor; a connection frame having one end connected to the support case and being extendable upward from the support case; and an action frame connected to the other end of the connection frame and on which a near-infrared lamp assembly is installed, wherein the near-infrared lamp assembly includes a plurality of double-tube lamps arranged on the action frame, each of the double-tube lamps comprises a central tube accommodating a lamp unit, a light transmission tube surrounding the central tube in a spaced state, and a liquid flowing between the central tube and the light transmission tube.

## Description

### Technical Field

The present disclosure relates to a near-infrared body care device using a near-infrared lamp assembly, and more particularly, to a device comprising: a support case supported on a floor; a connection frame having one end connected to the support case and being extendable upward from the support case; and an action frame connected to the other end of the connection frame and on which a near-infrared lamp assembly is installed, wherein the near-infrared lamp assembly includes a plurality of double-tube lamps arranged on the action frame, each of the double-tube lamps comprises a central tube accommodating a lamp unit, a light transmission tube surrounding the central tube in a spaced state, and a liquid flowing between the central tube and the light transmission tube, and the support case comprises a cooling unit for receiving and cooling the liquid from the near-infrared lamp assembly, a liquid storage tank for accommodating the liquid cooled by the cooling unit, and a pump for receiving the liquid from the liquid storage tank and pumping it to the near-infrared lamp assembly.

### Background Art

In general, far-infrared and near-infrared radiation are infrared rays close to solar radiant heat, which do not heat the air but only heat objects to which heat is transferred as wavelengths, and are short-wavelength rays harmful to the human body, with wavelengths longer than visible light and lower energy, having the property of being converted to heat when absorbed by objects.

When such infrared rays are transmitted to the human body, the property of deeply penetrating the human body and being converted to heat is widely used in infrared therapy devices that enable treatment of affected areas such as muscles or wounds using near-infrared rays emitted from infrared lamps.

Such infrared radiation is representative radiant energy that directly transfers energy from a high-temperature object to a low-temperature object without a medium, and is widely used for medical purposes to transfer energy to human skin or deep tissues. In order to maximize the therapeutic effect using infrared radiation, wIRA (Water-filtered Infrared-A) forming a 760-1,400 nm band is typically generated using halogen lamps for irradiation.

Conventional near-infrared lamp assemblies mainly use asbestos around the heating element to block the high heat generated during heating, which causes asbestos powder to separate during long-term use, thereby not only being harmful to the human body but also causing environmental pollution problems.

In addition, conventional near-infrared healthcare devices have the characteristic that, due to the nature of near-infrared radiation, the energy density delivered to the user decreases sharply as the distance between the lamp and the user increases, and accordingly, among a plurality of lamps, a lamp that is far away from the user may fail to deliver sufficient near-infrared radiation to the user. The use of lamps with low irradiation efficiency acts as one of the causes of reducing the overall energy efficiency of the device because the amount of near-infrared radiation delivered to the user is small while the amount of power consumption is the same as other lamps.

Additionally, Korean Patent Publication No. 10-2010-0039317 discloses an infrared TDP thermotherapy device for irradiating far-infrared rays, comprising a housing having an open portion on one side, an infrared lamp seated inside the open portion of the housing and emitting infrared rays, a TDP plate radiating specific electromagnetic waves from the inside of the infrared lamp toward the open portion, and a radiation plate seated inside the open portion and formed as a curved surface so that infrared rays emitted from the infrared lamp diffuse toward the open portion from the inside of the TDP.

Furthermore, Korean Patent No. 10-1441811 discloses technology for a thermotherapy device capable of concentrating on raising the deep body temperature of a treatment target by irradiating the treatment target using a halogen lamp having an infrared wavelength while removing light in a long wavelength band that raises the body temperature near the skin of the treatment target.

Moreover, Korean Patent No. 10-1558790 discloses technology for an infrared lamp tube comprising a case made of a tube body forming a space portion therein, an infrared light-emitting unit having a plurality of halogen lamps arranged in the lengthwise direction at intervals inside the case, caps fitted on both sides of the case respectively, and a filter liquid made of water filled in the space portion of the case.

According to the above-mentioned prior art documents, wIRA is generated using the wavelength characteristics of halogen lamps as an infrared radiation source, and the radiant energy of the halogen lamp is configured to be high-output so that irradiation is performed on the body.

When treatment is performed using the halogen lamp, light emitted from the halogen lamp comes into contact with the user's face, especially the user's eyes, causing damage to the eyeball.

### Detailed Description of the Invention

### Technical Problems

The present disclosure has been invented to improve the above-mentioned problems, and the problems to be solved by the present disclosure are to provide a double-tube lamp accommodating a lamp unit that emits infrared rays inside, formed with a central tube and a light transmission tube with a certain gap between the inner and outer circumferences, with double-tube caps installed at both ends to allow liquid for refrigerant (for example, water) to be charged and flow into the gap between the central tube and the light transmission tube, and adopting a water filter method which is a filtration method using water as a medium, whereby far-infrared and mid-infrared rays among the infrared rays emitted from the lamp unit are blocked and only near-infrared rays are emitted to the outside of the double-tube lamp, thereby enabling various health effects on the body by allowing deep penetration into subcutaneous tissue when the near-infrared rays are irradiated on the skin, to provide a near-infrared lamp assembly and a near-infrared body care system using the near-infrared lamp assembly.

The problem to be solved by the present disclosure is to provide a near-infrared lamp assembly capable of adjusting the brightness ratio of near-infrared emission lamps and controllably supplying and circulating according to the water level of cooling water to constantly control the high temperature heat emitted from the lamps and to control temperature adjustment according to the installation position of the near-infrared emission lamps, and a near-infrared body care system using the near-infrared lamp assembly.

The problem to be solved by the present disclosure is to provide an energy-efficient near-infrared healthcare device capable of maintaining healthcare effects by near-infrared radiation while reducing power consumption compared to using all double-tube lamps by irradiating near-infrared radiation to the user using only double-tube lamps that can provide near-infrared radiation with high efficiency to the user's body among a plurality of double-tube lamps that irradiate near-infrared radiation.

The technical problems of the present disclosure are not limited to those mentioned above, and other technical problems not mentioned will be clearly understood by those skilled in the art from the description below.

### Technical Solution

According to an embodiment of the present disclosure, there is provided a near-infrared body care device comprising: a support case supported on a floor; a connection frame having one end connected to the support case and being extendable upward from the support case; and an action frame connected to the other end of the connection frame and on which a near-infrared lamp assembly is installed, wherein the near-infrared lamp assembly includes a plurality of double-tube lamps arranged on the action frame, each of the double-tube lamps comprises a central tube accommodating a lamp unit, a light transmission tube surrounding the central tube in a spaced state, and a liquid flowing between the central tube and the light transmission tube, and the support case comprises a cooling unit for receiving and cooling the liquid from the near-infrared lamp assembly, a liquid storage tank for accommodating the liquid cooled by the cooling unit, and a pump for receiving the liquid from the liquid storage tank and pumping it to the near-infrared lamp assembly.

According to an embodiment of the present disclosure, when viewed from a plan or side, the action frame has a shape bent toward the opposite side with respect to the position of the connection frame, and a plurality of double-tube lamps are installed on the side of the action frame opposite to the position of the connection frame, wherein a lamp installation reflection bracket bent along the bent shape is installed on the side of the action frame opposite to the position of the connection frame, and the plurality of double-tube lamps are installed side by side.

According to an embodiment of the present disclosure, the lamp installation reflection bracket has a container shape with an open lamp entry portion facing the double-tube lamps to accommodate the plurality of double-tube lamps, installation grooves are respectively formed in the opposite side flanges of the lamp installation reflection bracket into which first liquid flow tubes extending from both ends of the double-tube lamps are inserted, and elastic support pieces supporting the vicinity of both ends of the double-tube lamps are installed at the bottom of the lamp installation reflection bracket.

According to an embodiment of the present disclosure, when viewed from the side, distribution pipes bent along the bent shape are installed on the side of the action frame where the double-tube lamps are arranged, and when viewed from the front, the distribution pipes are respectively installed on the left and right with the double-tube lamps in between, and a plurality of second liquid flow tubes extending toward the first liquid flow tubes are formed on the inner surface of each distribution pipe, and flexible tubes are connected between the spaced first liquid flow tubes and second liquid flow tubes.

According to an embodiment of the present disclosure, a compressor and a condenser are installed at the bottom of the support case, a heat radiation fan is installed on a side of the support case, and the liquid storage tank and a pump for pumping liquid are installed inside the support case.

According to an embodiment of the present disclosure, the compressor and the condenser are sequentially arranged from front to rear while being surrounded by a protective frame having a channel-shaped cross-section, the heat radiation fan is arranged on the rear surface of the support case to face the condenser, and the liquid storage tank and the pump are mounted on the top of the protective frame.

According to an embodiment of the present disclosure, an evaporator constituting the cooling unit is installed inside the liquid storage tank.

According to an embodiment of the present disclosure, the pump sucks low-temperature liquid from the liquid storage tank and supplies it to the double-tube lamps, and high-temperature liquid discharged from the double-tube lamps is supplied to the liquid storage tank and cooled by the evaporator.

According to an embodiment of the present disclosure, the connection frame connected to the support case and the action frame connected to the other end of the connection frame are configured to be capable of horizontal rotation and elevation rotation.

According to an embodiment of the present disclosure, a first connection pipe and a second connection pipe, one end of which is connected to the distribution pipe, extend respectively on the left and right sides with respect to the connection frame in the action frame, and the other ends of the first connection pipe and the second connection pipe are respectively connected to the support case and connected to the liquid storage tank.

According to an embodiment of the present disclosure, the connection frame and the action frame are detachably installed, and the first connection pipe and the second connection pipe are detachably installed to the action frame.

According to an embodiment of the present disclosure, the near-infrared body care device further comprises: a smart band or smart watch that can be worn by a user to check health status; and a mobile communication terminal configured with an installed application so that the health status improved by the near-infrared body care device can be visually confirmed.

According to an embodiment of the present disclosure, a camera is installed on the action frame toward the user's head.

### Advantageous Effects

As described above, the present disclosure has the following effects.

Furthermore, by providing a double-tube lamp accommodating a lamp unit that emits infrared rays inside, formed with a central tube and a light transmission tube with a certain gap between the inner and outer circumferences, with double-tube caps installed at both ends to allow liquid for refrigerant (for example, water) to be charged and flow into the gap between the central tube and the light transmission tube, and adopting a water filter method which is a filtration method using water as a medium, far-infrared and mid-infrared rays among the infrared rays emitted from the lamp unit are blocked and only near-infrared rays are emitted to the outside of the double-tube lamp, thereby enabling various health effects on the body by allowing deep penetration into subcutaneous tissue when the near-infrared rays are irradiated on the skin.

In addition, the double-tube lamp is configured to surround the outside of the lamp unit, and the liquid for refrigerant is charged and flows into the gap, thereby cooling the high temperature heat emitted from the lamp unit during near-infrared treatment and preventing the high temperature heat emitted from the lamp unit from directly contacting the user's skin to prevent skin burns.

Furthermore, the present disclosure has the effect of adjusting the brightness ratio of near-infrared emission lamps and controllably supplying and circulating according to the water level of cooling water to constantly control the high temperature heat emitted from the lamps and enabling temperature adjustment according to the installation position of the near-infrared emission lamps.

In addition, during near-infrared treatment, the proliferation, metastasis, and recurrence of cancer cells can be suppressed and eliminated. NK cells secrete perforin protein to make holes in cancer cells and inject an enzyme called granzyme to eliminate cancer cells. NK cells have activation receptors and inhibition receptors to distinguish and attack normal cells and abnormal cells. In addition, they also play a role in activating T cells and B cells by secreting IFN-Y (interferon gamma) substance.

Furthermore, in irradiating near-infrared radiation to the user using a plurality of double-tube lamps, double-tube lamps capable of irradiating near-infrared radiation to the user with higher efficiency are determined based on the distance between each distance sensor and the user's body sensed by a plurality of distance sensors, and high power efficiency can be achieved by irradiating near-infrared radiation to the user using the determined double-tube lamps.

The effects of the present disclosure are not limited to the effects mentioned above, and other effects not mentioned will be clearly understood by those skilled in the art from the description of the claims.

### Brief Description of the Drawings

FIG. 1 is a configuration diagram illustrating a near-infrared lamp assembly according to the present disclosure.
FIG. 2 is a block diagram illustrating an embodiment of a near-infrared body care system using a near-infrared lamp assembly according to the present disclosure.
FIG. 3 is a front perspective view illustrating a portable near-infrared body care device according to the present disclosure.
FIG. 4 is a rear perspective view illustrating a portable near-infrared body care device according to an embodiment of the present disclosure with a portion of the support case removed.
FIG. 5 is a bottom perspective view showing the coupling structure of the action frame and the near-infrared lamp assembly in a portable near-infrared body care device according to an embodiment of the present disclosure.
FIG. 6 is a rear top perspective view showing the arrangement configuration and liquid piping connection structure inside the support case in a portable near-infrared body care device according to an embodiment of the present disclosure.
FIG. 7 is a front bottom perspective view showing the arrangement configuration and liquid piping connection structure inside the support case in a portable near-infrared body care device according to an embodiment of the present disclosure.
FIG. 8 is a front view showing the arrangement configuration inside the support case in a portable near-infrared body care device according to an embodiment of the present disclosure.
FIG. 9 is a front perspective view showing the evaporator structure arranged in the liquid storage tank in a portable near-infrared body care device according to an embodiment of the present disclosure.
FIG. 10 is a view illustrating a blower installed at the lower side of the support case in a portable near-infrared body care device according to an embodiment of the present disclosure.
FIG. 11 is a block diagram illustrating the configuration of a cooling module in a portable near-infrared body care device according to an embodiment of the present disclosure.
FIG. 12 is a schematic diagram showing liquid piping, refrigerant piping, communication and electrical transmission configuration in a portable near-infrared body care device according to an embodiment of the present disclosure.
FIG. 13 is a block diagram illustrating that, in a portable near-infrared body care device according to an embodiment of the present disclosure, a biosensor unit detects user health information, receives the user health information detected by the biosensor unit through a smart band or smart watch to check user health status, and visually confirms the health status improved by near-infrared treatment of the near-infrared body care device through a wireless communication terminal.
FIGS. 14 and 15 are views for explaining the operation of a plurality of double-tube lamps according to an embodiment of the present disclosure.
FIG. 16 is a perspective view and a side view showing a lamp assembly including a circular lamp unit according to an embodiment of the present disclosure.
FIG. 17 is a perspective view showing a straight-type action frame and lamp assembly according to an embodiment of the present disclosure.
FIG. 18 is a perspective view showing a portable near-infrared body care device with a heat radiation fan disposed at the lower portion according to an embodiment of the present disclosure.
FIG. 19 is a perspective view illustrating a chamber-type near-infrared body care device according to another embodiment of the present disclosure.
FIG. 20 is a side view of FIG. 19.
FIG. 21 is a product photograph of a bed-type near-infrared body care device according to yet another embodiment of the present disclosure.
FIGS. 22 and 23 are perspective views of a bed-type near-infrared body care device according to yet another embodiment of the present disclosure.
FIG. 24 is a side view of a bed-type near-infrared body care device according to yet another embodiment of the present disclosure.
FIG. 25 is a front view of a bed-type near-infrared body care device according to yet another embodiment of the present disclosure.
FIG. 26 is a plan view illustrating an operation control unit in a bed-type near-infrared body care device according to yet another embodiment of the present disclosure.
FIG. 27 is an exploded perspective view of a bed-type near-infrared body care device according to yet another embodiment of the present disclosure.
FIG. 28 is a plan view for explaining the installation structure of a lamp unit in a bed-type near-infrared body care device according to yet another embodiment of the present disclosure.
FIG. 29 is an exploded perspective view for explaining the installation structure of a lamp unit in a bed-type near-infrared body care device according to yet another embodiment of the present disclosure.
FIG. 30 is a perspective view illustrating a main frame in a bed-type near-infrared body care device according to yet another embodiment of the present disclosure.
FIGS. 31 and 32 are views explaining the penetration rate of deep penetration into subcutaneous tissue when near-infrared radiation is irradiated on the skin during near-infrared treatment in a near-infrared body care system according to the present disclosure and the therapeutic effects on various diseases.

### Detailed Description

Hereinafter, a near-infrared lamp assembly according to a preferred embodiment of the present disclosure and a near-infrared body care system using the near-infrared lamp assembly will be described in detail with reference to the accompanying drawings.

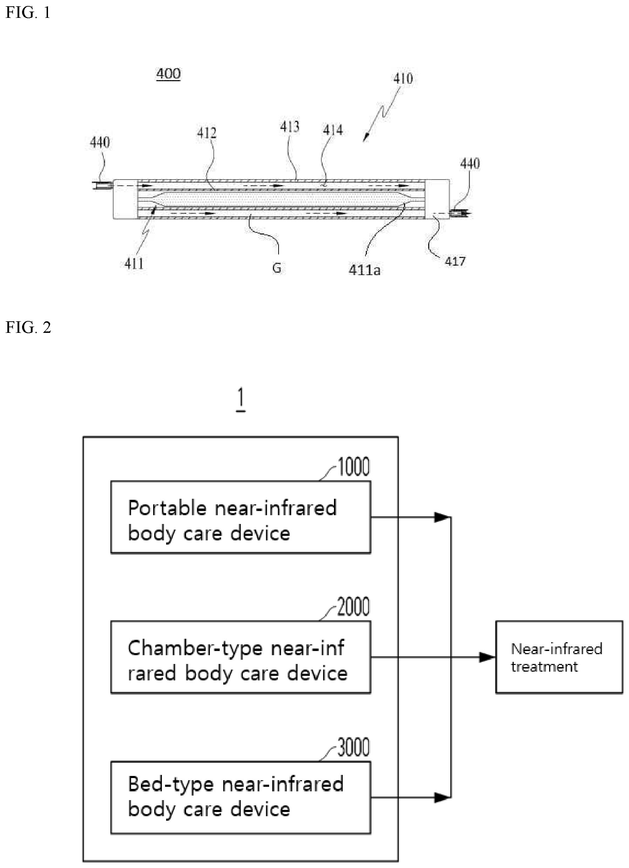

FIG. 1 is a configuration diagram illustrating a near-infrared lamp assembly according to the present disclosure.

As shown in FIG. 1, the near-infrared lamp assembly 400 according to the present disclosure comprises: a lamp unit 411 that emits infrared rays; and a double-tube lamp 410 accommodating the lamp unit 411 inside, formed with a central tube 412 and a light transmission tube 413 with a certain gap G between the inner and outer circumferences, and having double-tube caps 417 installed at both ends to allow liquid for refrigerant 414 to be charged and flow into the gap G between the central tube 412 and the light transmission tube 413.

The lamp unit 411 has a filament (not shown) installed inside a glass body 411a, and an inert gas (for example, nitrogen gas) is sealed inside the lamp unit 411.

The near-infrared lamp assembly 400 according to the present disclosure has the technical feature configured to block far-infrared and mid-infrared rays among the infrared rays emitted from the lamp unit 411 by the double-tube lamp structure, and emit only near-infrared rays to the outside of the double-tube lamp 410, thereby enabling deep penetration into subcutaneous tissue when the near-infrared rays are irradiated on the skin.

In the near-infrared lamp assembly 400 according to the present disclosure, the double-tube lamp 410 is arranged to surround the outside of the lamp unit 411, and the liquid for refrigerant is charged and flows into the gap G, thereby cooling the high temperature heat emitted from the lamp unit 411 during near-infrared treatment and preventing the high temperature heat emitted from the lamp unit 411 from directly contacting the user's skin to prevent skin burns while fully exhibiting the near-infrared therapeutic effect.

Furthermore, FIG. 2 is a block diagram illustrating an embodiment of a near-infrared body care system using a near-infrared lamp assembly according to the present disclosure.

As shown in FIG. 2, the near-infrared body care system 1 using a near-infrared lamp assembly according to the present disclosure has the technical feature of performing near-infrared treatment using the near-infrared lamp assembly 400 and comprising any one of a portable near-infrared body care device 1000, a chamber-type near-infrared body care device 2000, or a bed-type near-infrared body care device 3000.

That is, the near-infrared body care system 1 described may correspond to the portable near-infrared body care device 1000, the chamber-type near-infrared body care device 2000, or the bed-type near-infrared body care device 3000.

For convenience of explanation, in the case of the portable near-infrared body care device 1000, "portable" may be omitted and described as a near-infrared body care device 1000. Hereinafter, the (portable) near-infrared body care device 1000 of the present disclosure will be described.

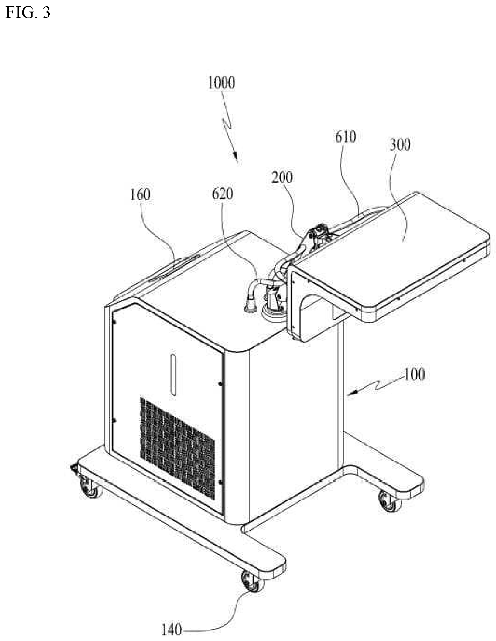

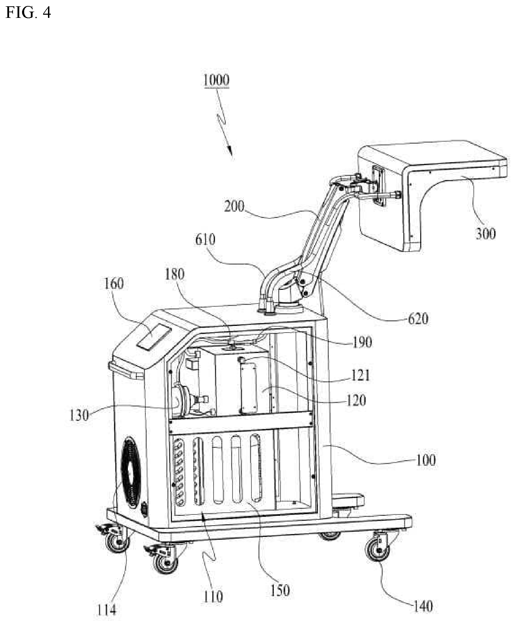

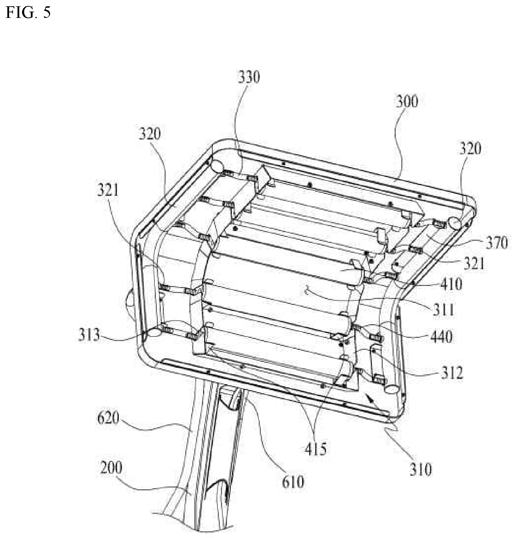

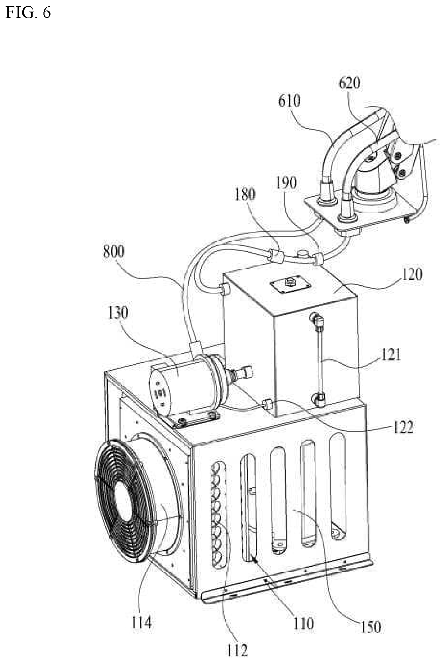

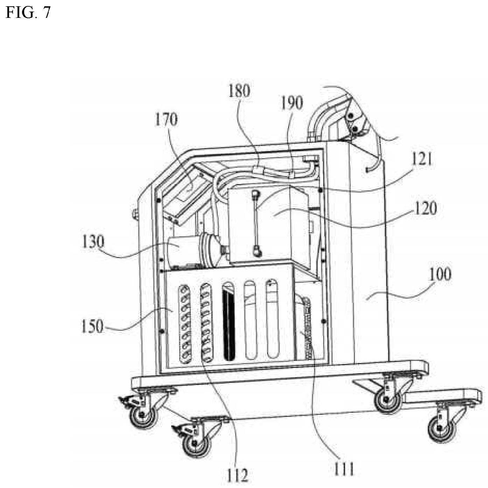

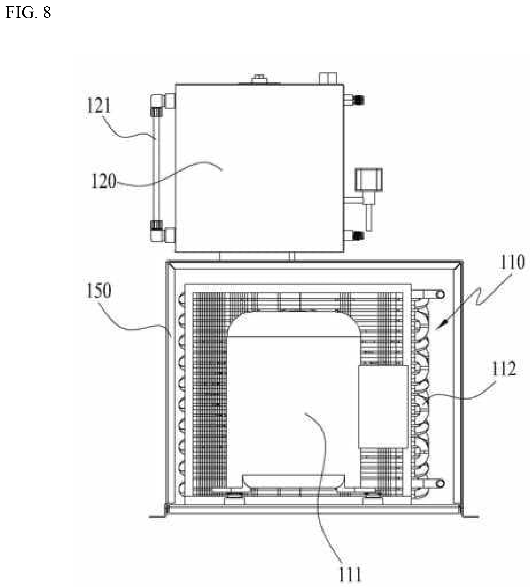

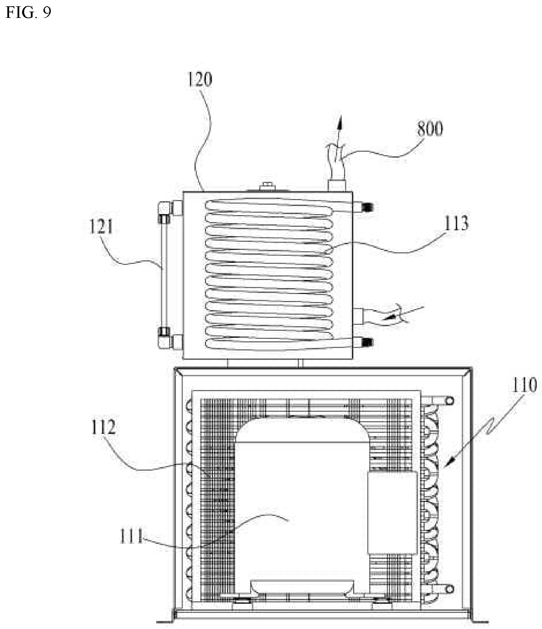

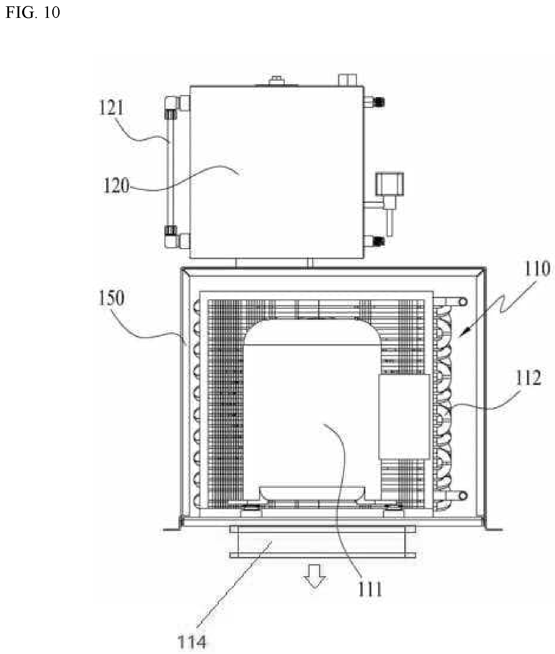

As shown in FIGS. 3 to 12, the near-infrared body care device 1000 of the present disclosure comprises: a support case 100 supported on a floor; a connection frame 200 having one end connected to the support case 100 and being extendable upward from the support case 100; and an action frame 300 connected to the other end of the connection frame 200 and provided with a near-infrared lamp assembly 400.

The near-infrared lamp assembly 400 comprises: a lamp unit 411 that emits infrared rays; and a double-tube lamp 410 accommodating the lamp unit 411 inside, formed with a central tube 412 and a light transmission tube 413 with a certain gap G between the inner and outer circumferences, and having double-tube caps 417 installed at both ends to allow liquid for refrigerant 414 to be charged and flow into the gap G between the central tube 412 and the light transmission tube 413.

That is, the near-infrared lamp assembly 400 includes a plurality of double-tube lamps 410 arranged on the action frame 300, each of the double-tube lamps 410 comprises a central tube 412 accommodating the lamp unit 411, a light transmission tube 413 surrounding the central tube 412 in a spaced state, and liquid 414 flowing between the central tube 412 and the light transmission tube 413, and the support case 100 comprises a cooling unit 110 for receiving and cooling the liquid 414 from the near-infrared lamp assembly 400, a liquid storage tank 120 for accommodating the liquid cooled by the cooling unit 110, and a pump 130 for receiving the liquid 414 from the liquid storage tank 120 and pumping it to the near-infrared lamp assembly 400.

The lamp unit 411 has a filament installed inside a glass body 411a, and an inert gas (for example, nitrogen gas) is sealed inside the lamp unit 411, and has the technical feature configured to block far-infrared and mid-infrared rays among the infrared rays emitted from the lamp unit 411 and emit only near-infrared rays to the outside of the double-tube lamp 410, thereby enabling deep penetration into subcutaneous tissue when the near-infrared rays are irradiated on the skin.

The double-tube lamp 410 is configured to surround the outside of the lamp unit 411, and the liquid for refrigerant is charged and flows into the gap G, thereby cooling the high temperature heat emitted from the lamp unit 411 and preventing the high temperature heat emitted from the lamp unit 411 from directly contacting the user's skin to effectively prevent skin burns.

The support case 100 includes a cooling module of a circulation and cooling method of liquid for refrigerant to cool the heat generation of the lamp unit 411, and the cooling module adopts a non-direct water supply method (refrigerant liquid self-circulation method) to facilitate movement and transportation of the near-infrared body care device.

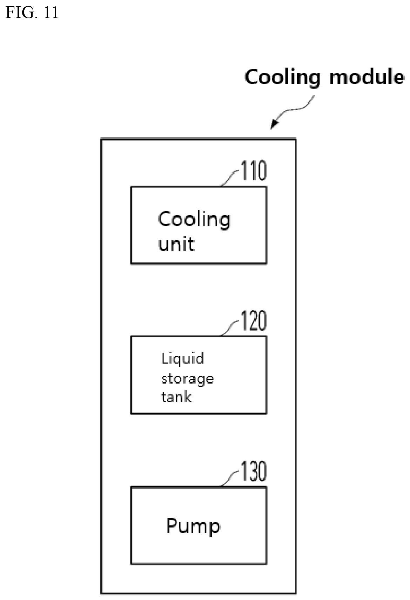

The cooling module, as shown in FIG. 11, comprises: a cooling unit 110 for receiving and cooling the liquid from the near-infrared lamp assembly 400; a liquid storage tank 120 for accommodating the liquid for refrigerant cooled by the cooling unit 110; and a pump 130 for receiving the liquid from the liquid storage tank 120 and pumping it toward the near-infrared lamp assembly 400.

The cooling module uses a water filter method to prevent contamination inside the liquid for refrigerant.

For reference, the present disclosure adopts a water filter method which is a filtration method using water as a medium, thereby playing a role in filtering unnecessary or harmful rays to the human body in the wavelength band of sunlight, and only the near-infrared (700-1400nm) wavelength extracted in this way can efficiently raise the deep body temperature without thermal damage to the skin.

Furthermore, in the (portable) near-infrared body care device 1000 of the present disclosure, when viewed from a plan or side, the action frame 300 has a shape bent toward the opposite side with respect to the position of the connection frame 200, and a plurality of double-tube lamps 410 are installed on the side of the action frame 300 opposite to the position of the connection frame 200.

In the action frame 300, a lamp installation reflection bracket 310 bent along the bent shape is installed on the side opposite to the position of the connection frame 200, and the plurality of double-tube lamps 410 are installed side by side.

Furthermore, the lamp installation reflection bracket 310 has a container shape with an open lamp entry portion 311 facing the double-tube lamps 410 to accommodate the plurality of double-tube lamps 410, installation grooves are respectively formed in the opposite side flanges 312 of the lamp installation reflection bracket 310 into which first liquid flow tubes 440 extending from both ends of the double-tube lamps 410 are inserted, and elastic support pieces 415 supporting the vicinity of both ends of the double-tube lamps 410 are installed at the bottom of the lamp installation reflection bracket 310.

The elastic support pieces 415 are respectively disposed on both sides with the double-tube lamp 410 in between and extend toward the lamp entry portion 311, and have a shape in which the waist portion is narrowly bent inward, so that the double-tube lamp 410 can be easily elastically coupled to the elastic support pieces 415 in a one-touch manner through the lamp entry portion 311 and can be easily separated.

Furthermore, in the near-infrared body care device 1000 of the present disclosure, when viewed from the side, the action frame 300 is provided with distribution pipes 320 bent along the bent shape on the side where the double-tube lamps 410 are arranged, and when viewed from the front, the distribution pipes 320 are respectively installed on the left and right with the double-tube lamps 410 in between, a plurality of second liquid flow tubes 321 extending toward the first liquid flow tubes 440 are formed on the inner surface of each distribution pipe 320, and flexible tubes 330 are connected between the spaced first liquid flow tubes 440 and second liquid flow tubes 321. The flexible tubes 330 may be made of rubber, silicone, or various other flexible and durable materials.

According to this configuration, even if the first liquid flow tubes 440 and the second liquid flow tubes 321 are not accurately arranged in a straight line, there is an advantage that a firm connection is possible without leakage by the flexible tubes 330.

The distribution pipes 320 may be installed on appropriate brackets 370 fixed on the action frame 300.

The support case 100 accommodates a cooling unit 110 for receiving and cooling the liquid 414 from the near-infrared lamp assembly 400, a liquid storage tank 120 for accommodating the liquid 414 cooled by the cooling unit 110, and a pump 130 for receiving the liquid 414 from the liquid storage tank 120 and pumping it to the near-infrared lamp assembly 400.

The cooling unit 110 serves to prevent the liquid 414 heated and circulated in the near-infrared lamp assembly 400 from continuously rising in temperature so that the liquid 414 can properly function as a near-infrared filter as well as maintaining the durability of the double tube, and consists of components constituting a general cooling cycle such as a compressor 111, a condenser 112, an expansion valve (not shown, integrated configuration), and an evaporator 113.

Furthermore, a compressor 111 and a condenser 112 are installed at the bottom of the support case 100, a heat radiation fan 114 is installed on a side or lower surface of the support case 100 to quickly radiate heat generated from the condenser 112, and a liquid storage tank 120 and a pump 130 for pumping the liquid 414 may be installed inside the support case 100.

Furthermore, the compressor 111 and the condenser 112 are sequentially arranged from front to rear while being surrounded by a protective frame 150 having a channel-shaped cross-section, the heat radiation fan 114 is arranged on the rear surface of the support case 100 to face the condenser 112, and the liquid storage tank 120 and the pump 130 are mounted on the top of the protective frame 150.

Furthermore, an evaporator 113 constituting the cooling unit 110 is installed inside the liquid storage tank 120. The evaporator 113 may correspond to a coil shape wound upward while being supported at the bottom of the liquid storage tank 120, and accordingly, since the evaporator 113 is not separately installed outside, the floor area occupied by the support case 100 can be further reduced. Of course, a thermoelectric element may be adopted as the cooling unit 110 to block noise generated from the compressor 111.

Furthermore, the pump 130 sucks low-temperature liquid from the liquid storage tank 120 and supplies it to the double-tube lamps 410, and the high-temperature liquid discharged from the double-tube lamps 410 is resupplied to the liquid storage tank 120 and cooled by the evaporator 113. That is, it is structured so that the evaporator 113 can directly cool the liquid in the liquid storage tank 120.

In this case, it is preferable that the distance between the point where the liquid is returned to the liquid storage tank 120 and the point where the low-temperature liquid is discharged is spaced apart as much as possible so that the liquid sufficiently cooled by the evaporator 113 flows to the pump 130.

For example, as shown, the evaporator 113 may be installed upright in the liquid storage tank 120, the point where the liquid flows in may be disposed inside the evaporator 113 at the bottom of the liquid storage tank 120, and the point where it is discharged may be disposed on the upper side surface of the liquid storage tank 120.

Furthermore, the high-temperature liquid introduced from the bottom of the liquid storage tank 120 is uniformly cooled while rising as a spiral (vortex) flow is generated by the evaporator 113, and may be discharged through the upper side surface of the liquid storage tank 120 toward the pump 130.

Furthermore, the connection frame 200 connected to the support case 100 and the action frame 300 connected to the other end of the connection frame 200 are configured to be capable of horizontal rotation and elevation rotation, so that it is possible to freely irradiate near-infrared radiation to any part of the user's head, face, and abdomen.

Furthermore, a first connection pipe 610 and a second connection pipe 620, one end of which is connected to the distribution pipe 320, extend respectively on the left and right sides with respect to the connection frame 200 in the action frame 300, and the other ends of the first connection pipe 610 and the second connection pipe 620 are respectively connected to the support case 100 and may be connected to the liquid storage tank 120.

Furthermore, the connection frame 200 and the action frame 300 are detachably installed, and the first connection pipe 610 and the second connection pipe 620 are detachably installed to the action frame 300, so that they can be replaced with various types of action frames 300 according to the body part to be irradiated and used for multiple purposes.

Furthermore, a water level sensor 121 for detecting the liquid level in the liquid storage tank 120 is installed to issue a warning message when it falls below or exceeds an appropriate water level range and may be configured to enable emergency stop.

Furthermore, a temperature sensor 122 for detecting the internal liquid temperature is installed in the liquid storage tank 120 to detect the liquid temperature so that it is within an appropriate temperature range.

A touch screen 160 is installed on one side of the upper surface of the support case 100 to enable operation or emergency operation stop of the device, irradiation temperature adjustment, etc., and the liquid temperature, water level, compressor temperature, irradiation temperature, etc. of the liquid storage tank 120 may be displayed.

Furthermore, it is preferable that a main controller (PCB) 170 is installed on the inner surface opposite to the touch screen 160 in the support case 100 to improve space efficiency. In particular, it is preferable that the touch screen 160 and the main controller 170 are installed on the rear surface of the support case 100 on the side where the heat radiation fan 114 is installed to avoid exposure to high temperatures.

Reference numeral 180 not described above denotes a check valve and 190 denotes a flow switch. If the flow switch 190 does not detect flow during operation, it is determined that the device is abnormal and an emergency operation stop function is performed so that maintenance can be performed.

Furthermore, reference numeral 800 denotes a liquid connection pipe connecting the pump 130, the near-infrared lamp assembly 400, and the liquid storage tank 120. It is preferable that wheels 140 are installed at the bottom of the support case 100 to enable easy transportation and movement.

Although the above-described embodiment has been described mainly for the user's head and face, it may be equally applied to various parts such as the abdomen, chest, or pelvis of a lying user.

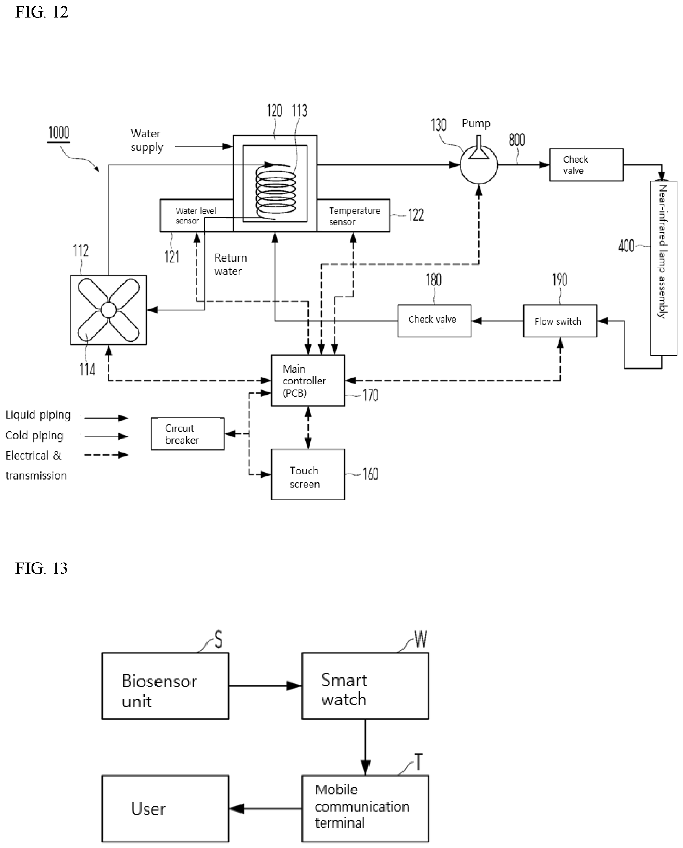

FIG. 12 shows an example of components, liquid piping, refrigerant piping, communication and electrical connection configuration constituting the near-infrared body care device 1000. First, when the near-infrared body care device 1000 is operated through the touch screen 160, the liquid in the liquid storage tank 120 is moved to the near-infrared lamp assembly 400 by the pump 130.

The lamp unit 411 in the double-tube lamp 410 constituting the near-infrared lamp assembly 400 is turned on to perform irradiation, wherein mid-infrared and far-infrared rays are filtered by the liquid flowing through the double-tube lamp 410 and only near-infrared rays escape.

The liquid heated by the lamp unit 411 is discharged from the double-tube lamp 410 and directed to the liquid storage tank 120 for cooling. In this case, since a coil-type evaporator 113 constituting the cooling unit 110 is installed inside the liquid storage tank 120, the liquid is rapidly cooled and the cooled liquid flows again to the double-tube lamp 410 through the pump 130.

By such a circulation method, continuous near-infrared irradiation is performed on various parts such as the user's head, abdomen, or pelvis.

Furthermore, as shown in FIG. 13, in the portable near-infrared body care device 1000 of the present disclosure, a biosensor unit S that detects user health information; a smart band or smart watch W that receives the user health information detected by the biosensor unit and that can be worn by the user to check health status; and a mobile communication terminal T configured with an installed application so that the health status improved by near-infrared treatment of the near-infrared body care device can be visually confirmed; are further included.

The biosensor unit S is installed inside the action frame 300 to photograph the user's body, for example, the head portion, and can precisely check the user's health status based on artificial intelligence.

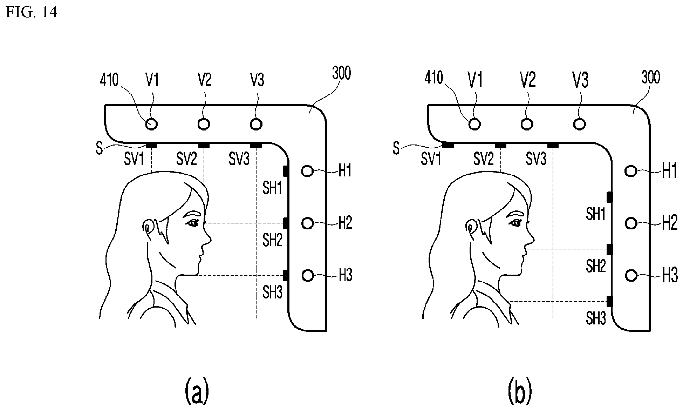

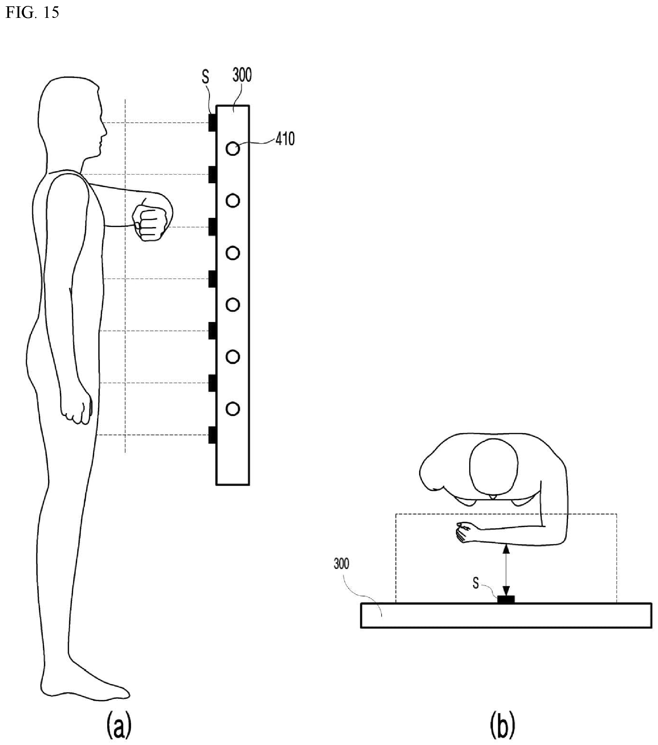

FIGS. 14 and 15 are views for explaining the operation of a plurality of double-tube lamps according to an embodiment of the present disclosure.

The near-infrared body care device 1000 according to an embodiment of the present disclosure can save energy by irradiating near-infrared radiation to the user using only at least one double-tube lamp 410 with excellent near-infrared transmission effect in irradiating near-infrared radiation to the user using a plurality of double-tube lamps 410. To this end, the head frame 300 according to an embodiment of the present disclosure may be provided with a plurality of distance sensors S. Each distance sensor S is arranged to detect the position of an object located in front of the head frame 300 front surface, thereby detecting the distance between the distance sensor S and the user's body and transmitting it to the main controller 170. The main controller 170 determines a double-tube lamp 410 capable of irradiating near-infrared radiation to the user with relatively high efficiency among the plurality of double-tube lamps 410 based on the distance information received from each distance sensor S, and improves power efficiency by turning on the determined double-tube lamp 410.

In this embodiment, the distance sensor S may be an ultrasonic sensor, but is not limited thereto. Hereinafter, the operation of the main controller 170 will be examined with reference to FIG. 14 explaining the operation of a plurality of double-tube lamps 410 in an embodiment in which the head frame 300 is formed in a bent shape and FIG. 15 explaining the operation of a plurality of double-tube lamps 410 in an embodiment in which the head frame 300 is formed in a flat plate shape.

FIG. 14(a) shows each distance sensor SV1, SV2, SV3, SH1, SH2, SH3 installed in line with each double-tube lamp V1, V2, V3, H1, H2, H3. More specifically, FIG. 14(a) shows each distance sensor SV1, SV2, SV3, SH1, SH2, SH3 installed on a plane perpendicular to the front surface of the head frame 300 and passing through the center line of each double-tube lamp 410. In an embodiment of the present disclosure, each distance sensor SV1, SV2, SV3, SH1, SH2, SH3 is installed on the surface of the light transmission tube 413 of each double-tube lamp 410, and can detect a distance in a direction perpendicular to the front surface of the head frame 300. In this case, considering that the user generally places the body near the center of the width of the head frame 300, it is preferable that each distance sensor SV1, SV2, SV3, SH1, SH2, SH3 is installed at the middle point of the length of each double-tube lamp 410 for more accurate detection of the user's position.

In another embodiment, each distance sensor SV1, SV2, SV3, SH1, SH2, SH3 may be installed on one side or both sides in the longitudinal direction of each double-tube lamp 410. At this time, each distance sensor SV1, SV2, SV3, SH1, SH2, SH3 is installed in an area of the front surface of the head frame 300 where the reflection bracket 310 is not installed, thereby minimizing heat transferred from the reflection bracket 310.

Each distance sensor SV1, SV2, SV3, SH1, SH2, SH3 detects the distance between each distance sensor SV1, SV2, SV3, SH1, SH2, SH3 and an object located in front of the front surface of the head frame 300 and transmits it to the main controller 170. The main controller 170 compares the distance value between each distance sensor SV1, SV2, SV3, SH1, SH2, SH3 and the object received from each distance sensor SV1, SV2, SV3, SH1, SH2, SH3 with a preset threshold distance. As a result of the comparison, the double-tube lamp 410 matched with each distance sensor SV1, SV2, SV3, SH1, SH2, SH3 at which a distance less than or equal to the threshold distance is measured is turned on to emit near-infrared radiation.

In this way, by comparing the threshold distance value with the actual distance value detected by each distance sensor SV1, SV2, SV3, SH1, SH2, SH3 and turning the double-tube lamp 410 on and off, the main controller 170 can prevent the distance sensor SV1, SV2, SV3, SH1, SH2, SH3 from detecting an object other than the user's body, such as the floor or side wall of the space where the user is located, as the user's body and controlling the on/off of the double-tube lamp 410.

In an embodiment of the present disclosure, each distance sensor SV1, SV2, SV3, SH1, SH2, SH3 and the double-tube lamp 410 may be matched one-to-one. For example, each distance sensor SV1, SV2, SV3, SH1, SH2, SH3 may be matched one-to-one with the double-tube lamp 410 located in line with each distance sensor SV1, SV2, SV3, SH1, SH2, SH3. Therefore, distance sensor SV1 is matched with double-tube lamp V1, distance sensor SV2 is matched with double-tube lamp V2, distance sensor SV3 is matched with double-tube lamp V3, distance sensor SH1 is matched with double-tube lamp H1, distance sensor SH2 is matched with double-tube lamp H2, and distance sensor SH3 is matched with double-tube lamp H3, respectively.

Therefore, as shown in FIG. 14(a), among the plurality of distance sensors SV1, SV2, SV3, SH1, SH2, SH3, when the distance to the user's body is detected to be less than or equal to the threshold distance by the distance sensors SV1, SV2, SH1, SH2, SH3, and the distance to the user's body is detected to exceed the threshold distance by the distance sensor SV3, the main controller 170 emits near-infrared radiation using only the double-tube lamps V1, V2, H1, H2, H3, and the double-tube lamp V3 does not emit near-infrared radiation. This is because the double-tube lamp V3 is not positioned relatively close to the user's body and cannot irradiate sufficient near-infrared radiation to the user, and thus the double-tube lamp 410 with low near-infrared efficiency applied to the user compared to power consumption maintains an off state, thereby improving power efficiency.

In another embodiment of the present disclosure, each distance sensor SV1, SV2, SV3, SH1, SH2, SH3 and the double-tube lamp 410 may be matched one-to-many. For example, each distance sensor SV1, SV2, SV3, SH1, SH2, SH3 may be matched with the double-tube lamp 410 located in line with each distance sensor SV1, SV2, SV3, SH1, SH2, SH3 and the double-tube lamp 410 located adjacent thereto. Therefore, distance sensor SV1 is matched with double-tube lamp V1 and double-tube lamp V2, distance sensor SV2 is matched with double-tube lamp V2, double-tube lamp V1 and double-tube lamp V3, distance sensor SV3 is matched with double-tube lamp V3 and double-tube lamp V2, distance sensor SH1 is matched with double-tube lamp H1 and double-tube lamp H2, distance sensor SH2 is matched with double-tube lamp H2, double-tube lamp H1 and double-tube lamp H3, and distance sensor SH3 is matched with double-tube lamp H3 and double-tube lamp H2, respectively.

Therefore, as shown in FIG. 14(a), among the plurality of distance sensors SV1, SV2, SV3, SH1, SH2, SH3, when the distance to the user's body is detected to be less than or equal to the threshold distance by the distance sensors SV1, SV2, SH1, SH2, SH3, and the distance to the user's body is detected to exceed the threshold distance by the distance sensor SV3, the main controller 170 can turn on the double-tube lamps V1, V2, V3, H1, H2, H3 to emit near-infrared radiation. That is, the main controller 170 causes all double-tube lamps 410 to irradiate near-infrared radiation to the user. In this way, when each distance sensor SV1, SV2, SV3, SH1, SH2, SH3 and the double-tube lamp 410 are matched one-to-many, power efficiency may be somewhat reduced, but more near-infrared radiation can be irradiated to the user compared to when each distance sensor SV1, SV2, SV3, SH1, SH2, SH3 and the double-tube lamp 410 are matched one-to-one, thereby increasing the user's perceived effect.

In an embodiment of the present disclosure, the threshold distance, which is the criterion for determining on/off of each double-tube lamp 410, may be set differently depending on the installation position of each distance sensor S. As shown in FIG. 14(a), when the head frame 300 is bent, the plurality of distance sensors SV1, SV2, SV3, SH1, SH2, SH3 can be divided into vertical distance sensors SV1, SV2, SV3 that detect objects below based on the front surface of the head frame 300 and horizontal distance sensors SH1, SH2, SH3 that detect objects on the side. At this time, the main controller 170 turns on the double-tube lamp 410 matched with each vertical distance sensor SV1, SV2, SV3 if the vertical distance detected by the vertical distance sensor SV1, SV2, SV3 is less than or equal to the vertical threshold distance, and turns on the double-tube lamp 410 matched with each horizontal distance sensor SH1, SH2, SH3 if the horizontal distance detected by the horizontal distance sensor SH1, SH2, SH3 is less than or equal to the horizontal threshold distance. In this embodiment, the vertical threshold distance and the horizontal threshold distance may be set differently.

In this embodiment, the vertical threshold distance may be equal to the distance between the plane passing through the lowermost end of the head frame 300 and parallel to the front surface of the head frame 300 on which each vertical distance sensor SV1, SV2, SV3 is installed and each vertical distance sensor SV1, SV2, SV3. Meanwhile, the horizontal threshold distance may be equal to the distance between the plane passing through the outermost side of the head frame 300 and parallel to the front surface of the head frame 300 on which each horizontal distance sensor SH1, SH2, SH3 is installed and each horizontal distance sensor SH1, SH2, SH3. That is, the main controller 170 can turn on at least one double-tube lamp 410 only when the user's body is located within a space in which two surfaces are defined by the front surface of the bent head frame 300. In this way, setting different threshold distances for the vertical distance sensors SV1, SV2, SV3 and the horizontal distance sensors SH1, SH2, SH3 is to be able to correspond to various shapes of the head frame 300.

FIG. 14(b) shows a state in which each distance sensor SV1, SV2, SV3, SH1, SH2, SH3 is positioned staggered with each double-tube lamp V1, V2, V3, H1, H2, H3 according to another embodiment of the present disclosure. Each distance sensor SV1, SV2, SV3, SH1, SH2, SH3 may be disposed spaced apart from each double-tube lamp 410 in the circumferential direction of each double-tube lamp 410. Since the detection of objects by each distance sensor SV1, SV2, SV3, SH1, SH2, SH3 and the on/off operation of the double-tube lamp 410 according to this embodiment are similar to those examined with reference to FIG. 14(a), the following description will focus on the differences to prevent the description from becoming complicated.

In an embodiment of the present disclosure, each distance sensor SV1, SV2, SV3, SH1, SH2, SH3 is disposed on the front surface of the reflection bracket 310 in the circumferential direction of each double-tube lamp 410, and can detect a distance in a direction perpendicular to the front surface of the head frame 300. In this case, considering that the user generally places the body near the center of the width of the head frame 300, it is preferable that each distance sensor SV1, SV2, SV3, SH1, SH2, SH3 is installed on the reflection bracket 310 at the middle point of the width of the head frame 300 for more accurate detection of the user's position. Of course, it is also possible for each distance sensor SV1, SV2, SV3, SH1, SH2, SH3 to be installed outside the reflection bracket 310 to receive minimal heat from the reflection bracket 310.

In an embodiment of the present disclosure, each distance sensor SV1, SV2, SV3, SH1, SH2, SH3 and the double-tube lamp 410 may be matched one-to-one. For example, distance sensor SV1 may be matched with double-tube lamp V1, distance sensor SV2 may be matched with double-tube lamp V2, distance sensor SV3 may be matched with double-tube lamp V3, distance sensor SH1 may be matched with double-tube lamp H1, distance sensor SH2 may be matched with double-tube lamp H2, and distance sensor SH3 may be matched with double-tube lamp H3, respectively.

Therefore, as shown in FIG. 14(b), among the plurality of distance sensors SV1, SV2, SV3, SH1, SH2, SH3, when the distance to the user's body is detected to be less than or equal to the threshold distance by the distance sensors SV1, SV2, SH1, SH2, SH3, and the distance to the user's body is detected to exceed the threshold distance by the distance sensor SV3, the main controller 170 emits near-infrared radiation using only the double-tube lamps V1, V2, H1, H2, H3, and the double-tube lamp V3 does not emit near-infrared radiation. This is because the double-tube lamp V3 is not positioned relatively close to the user's body and cannot irradiate sufficient near-infrared radiation to the user, and thus the double-tube lamp 410 with low near-infrared efficiency applied to the user compared to power consumption maintains an off state, thereby improving power efficiency.

In another embodiment of the present disclosure, some of the plurality of distance sensors SV1, SV2, SV3, SH1, SH2, SH3 may be matched one-to-one with the double-tube lamp 410, and the remaining some may be matched one-to-two with the double-tube lamp 410. For example, among the plurality of distance sensors SV1, SV2, SV3, SH1, SH2, SH3, distance sensors S not disposed between two double-tube lamps 410, that is, distance sensor SV1 and distance sensor SH3 are matched one-to-one with double-tube lamp V1 and double-tube lamp H3, respectively. However, the remaining distance sensors S disposed between two double-tube lamps 410, that is, distance sensor SV2, distance sensor SV3, distance sensor SH1 and distance sensor SH2 are respectively matched with two adjacent double-tube lamps 410. In other words, distance sensor SV2 is matched with double-tube lamp V1 and double-tube lamp V2, distance sensor SV3 is matched with double-tube lamp V2 and double-tube lamp V3, distance sensor SH1 is matched with double-tube lamp H1 and double-tube lamp H2, and distance sensor SH2 is matched with double-tube lamp H2 and double-tube lamp H3.

Therefore, as shown in FIG. 14(b), among the plurality of distance sensors SV1, SV2, SV3, SH1, SH2, SH3, when the distance to the user's body is detected to be less than or equal to the threshold distance by the distance sensors SV1, SV2, SH1, SH2, SH3, and the distance to the user's body is detected to exceed the threshold distance by the distance sensor SV3, the main controller 170 emits near-infrared radiation using only the double-tube lamps V1, V2, H1, H2, H3, and the double-tube lamp V3 does not emit near-infrared radiation. This is because the double-tube lamp V3 is not positioned relatively close to the user's body and cannot irradiate sufficient near-infrared radiation to the user, and thus the double-tube lamp 410 with low near-infrared efficiency applied to the user compared to power consumption maintains an off state, thereby improving power efficiency.

Meanwhile, it is also possible for the user to change his or her position relative to the head frame 300 while being exposed to near-infrared radiation irradiated from the head frame 300. For example, the user may move forward toward the head frame 300 to receive near-infrared radiation more closely, and may move backward away from the head frame 300 by feeling heat on the skin. In order to operate the plurality of double-tube lamps 410 more effectively in accordance with such user movement, the main controller 170 can control the on/off of each double-tube lamp 410 based on a change in the distance sensing state by each distance sensor S.

The main controller 170 monitors the distance detected from each distance sensor S in real time during operation of the near-infrared device 1000. As a result of monitoring, when a change in the distance detection state by any one distance sensor S is maintained for more than a first threshold time, the on/off state of at least one double-tube lamp 410 matched with any one distance sensor is changed. For example, if a distance sensor S that detected the distance to the user as less than or equal to the threshold distance detects that the distance to the user exceeds the threshold distance, and this detection state is maintained for more than the first threshold time, the main controller 170 can turn off the double-tube lamp 410 matched with the corresponding distance sensor S.

On the other hand, if a distance sensor S that detected the distance to the user as exceeding the threshold distance detects that the distance to the user is less than or equal to the threshold distance, and this detection state is maintained for more than the first threshold time, the main controller 170 can turn on the double-tube lamp 410 matched with the corresponding distance sensor S. In this way, by automatically controlling the operation of the plurality of double-tube lamps 410 in accordance with the user's movement, the effect by near-infrared radiation can be improved, and user satisfaction can also be increased.

Meanwhile, at the start of operation of the near-infrared device 1000, the main controller 170 can recognize the user's initial position and efficiently turn on/off the plurality of double-tube lamps 410. The main controller 170 according to this embodiment turns on the double-tube lamp 410 mapped to the distance sensor S where a distance less than or equal to the threshold distance is detected for more than a second threshold time within a measurement reference time when power is input to the near-infrared device 1000 at the user's position.

For example, when the measurement reference time is 3 seconds and the second threshold time is 2 seconds, the main controller 170 turns on the double-tube lamp 410 matched with the distance sensor S that detected a distance less than or equal to the threshold distance for a total of 2 seconds or more during 3 seconds after power is turned on. At this time, the second threshold time does not need to be a continuous time and is calculated as a total time. The reason for cumulatively measuring the threshold time that is discontinuously determined by setting a measurement reference time at the start of operation of the near-infrared device 1000, that is, discrete time, is that during initial operation of the near-infrared device 1000, the user often moves to adjust posture or touch the device for operation of the near-infrared device 1000, and therefore continuous time measurement may not reflect the user's intention.

FIG. 15 is a view for explaining the operation of a plurality of double-tube lamps 410 in an embodiment in which the head frame 300 is formed in a flat plate shape. In this embodiment, since the matching of each distance sensor S and double-tube lamp 410, distance detection by each distance sensor S, and on/off of the double-tube lamp 410 based thereon are the same as described with reference to FIG. 14, the following description will focus on the differences.

As shown in FIG. 15, the head frame 300 may be formed in a flat plate shape without being bent. When the head frame 300 is formed in a flat plate shape in this way, each of the plurality of distance sensors S all detects the user located in front of the head frame 300 in the same direction. When all distance sensors S detect objects in the same direction in this way, the main controller 170 can set the same threshold distance for all distance sensors S.

In this way, by having each distance sensor S have the same threshold distance, the main controller 170 can irradiate near-infrared radiation with high efficiency to the user's body located inside a plane parallel to the front surface of the head frame 300 and spaced apart from the front surface of the head frame 300 by the threshold distance, thereby performing an operation that more conforms to the user's intended use.

According to the near-infrared device 1000 according to this embodiment, in irradiating near-infrared radiation to the user using a plurality of double-tube lamps 410, a double-tube lamp 410 capable of irradiating near-infrared radiation to the user with higher efficiency is determined based on the distance between each distance sensor and the user's body detected by a plurality of distance sensors S, and there is a technical effect of improving power efficiency by irradiating near-infrared radiation to the user using the determined double-tube lamp 410.

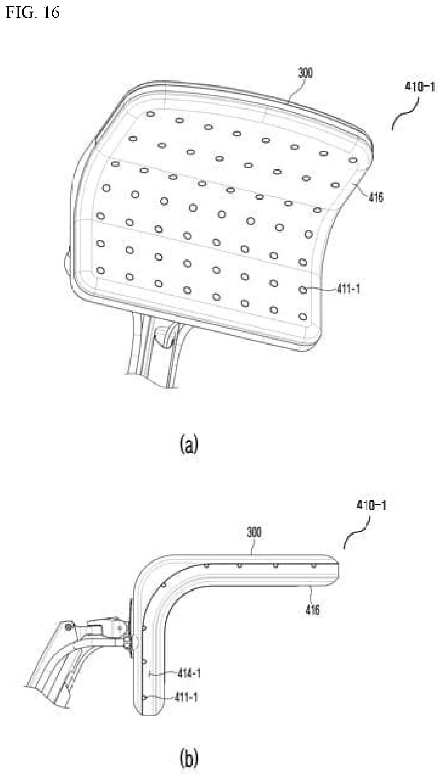

Meanwhile, the lamp unit 411 installed in the near-infrared body care device 1000 of the present disclosure is usually plural and aligned on the action frame 300, but in some cases, only one may be installed. Furthermore, as described above, the lamp unit 411 usually corresponds to a tube shape (double tube), but may also form a circular shape. Below, a circular lamp unit will be examined together with FIG. 16.

FIG. 16 is a perspective view and a side view showing a lamp assembly including a circular lamp unit according to an embodiment of the present disclosure.

According to an embodiment of the present disclosure, the lamp assembly 400 may correspond to a panel lamp 410-1 including a circular lamp unit 411-1. In FIG. 16, the panel lamp 410-1 includes a plurality of lamp units 411-1, but in some cases, only one lamp unit 411-1 may be included in the panel lamp 410-1, that is, the lamp assembly 400.

The lamp assembly 400 may include a circular lamp unit 411-1 and a filter unit. The filter unit may include a film 416 surrounding all of the plurality of lamp units 411-1, and a fluid 414-1 flowing around each of the plurality of lamp units 411-1 between the action frame 300 and the film 416. Here, the fluid 414-1 may correspond to liquid or gas.

Specifically, at least one lamp unit 411-1 is aligned on the action frame 300, and a fluid 414-1 may be positioned around the lamp unit 411-1 to cool the heat of the lamp unit 411-1 and emit only energy (near-infrared) having a wavelength in a certain range.

Furthermore, a film 416 may be installed on the action frame 300 to induce the fluid 414-1 to flow and prevent it from escaping. As can be seen in FIG. 14(b), the lamp unit 411-1 is installed on the action frame 300, and the fluid 414-1 may be positioned between the action frame 300 and the film 416.

Since the panel lamp 410-1 including the circular lamp unit 411-1 also has the same structure for the remaining systems (cooling system, etc.), description of common parts will be omitted.

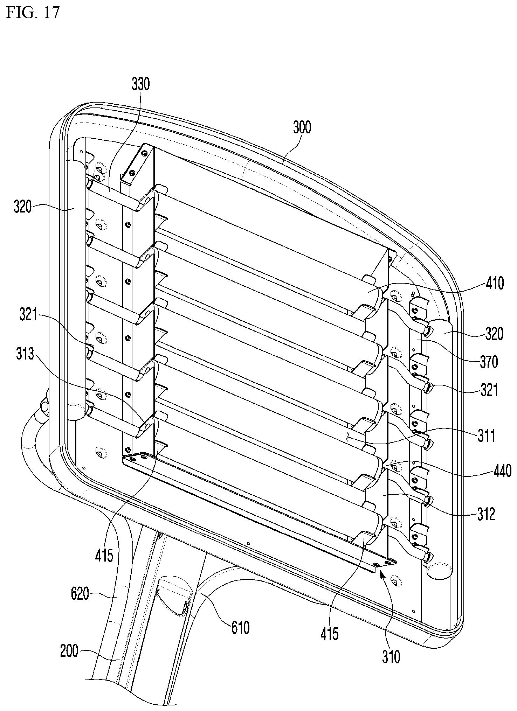

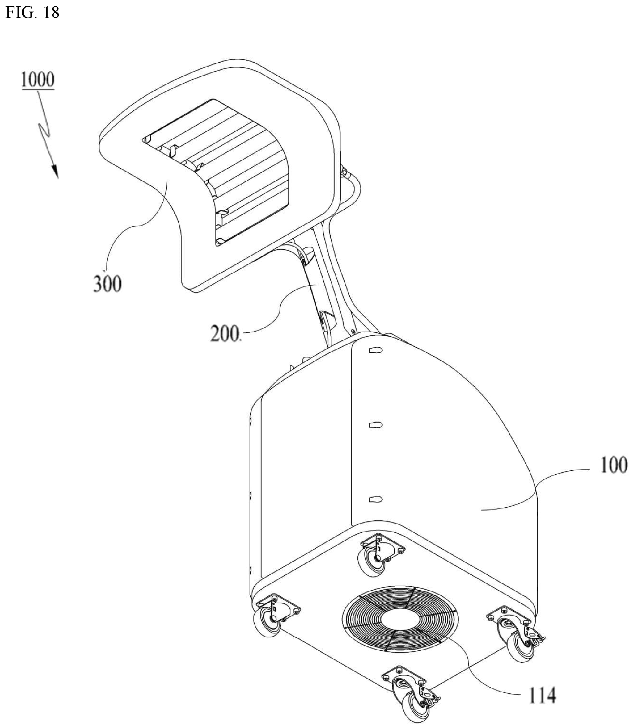

FIG. 17 is a perspective view showing a straight-type action frame and lamp assembly according to an embodiment of the present disclosure, and FIG. 18 is a perspective view showing a portable near-infrared body care device with a heat radiation fan disposed at the lower portion according to an embodiment of the present disclosure.

Meanwhile, although the above-described action frame 300 is shown in an L-shaped bent form, it is not limited thereto and may be bent in various other forms.

As can be seen in FIG. 17, the near-infrared body care device 1000 of the present disclosure may include an action frame 300 in a straight form without being bent. Only the form is different, and functionally it will be the same as the L-shaped bent form.

Furthermore, when viewed from a plan, it is also possible to bend in a wide U-shape toward the opposite side with respect to the position of the connection frame 200.

Furthermore, the above-described heat radiation fan 114 does not necessarily need to be located at the rear, and may be located at the bottom of the support case 100 as in FIG. 18. In this case, it may be more advantageous in terms of cooling performance or space utilization.

Hereinafter, the chamber-type near-infrared body care device 2000 of the present disclosure will be described.

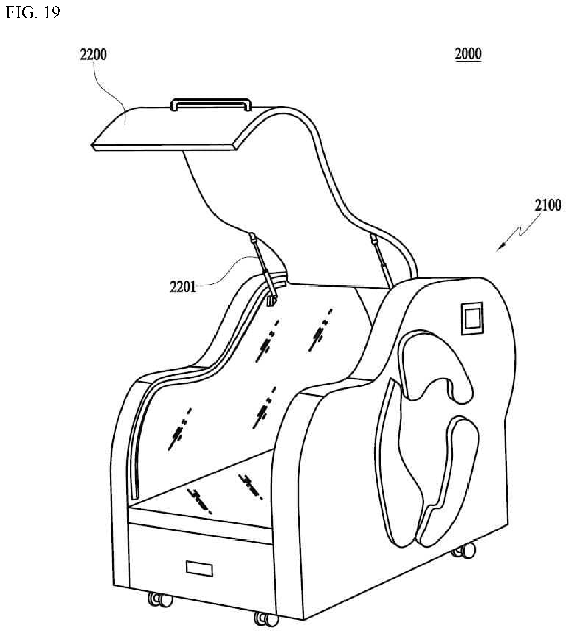

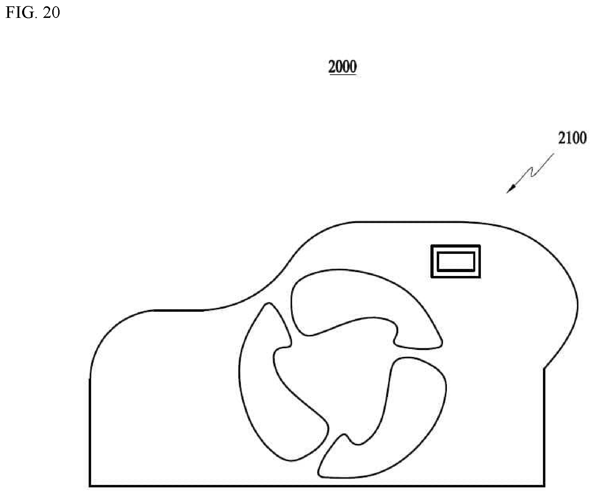

FIG. 19 is a perspective view illustrating a chamber-type near-infrared body care device according to another embodiment of the present disclosure, and FIG. 20 is a side view of FIG. 19.

Referring to FIGS. 19 and 20, the chamber-type near-infrared body care device 2000 of the present disclosure comprises a base body 2100 supported on the floor and having a space portion where the user can lie down; a cover 2200 for opening and closing the base body 2100; and a near-infrared lamp assembly 400 (shown in FIG. 1) installed inside the base body 2100 and configured so that far-infrared and mid-infrared rays among the emitted infrared rays are blocked and only near-infrared rays are emitted to penetrate deeply into subcutaneous tissue when the near-infrared rays are irradiated on the skin.

The cover 2200 may be configured to be stably opened and closed by a shock absorber 2201.

The base body 2100 includes a cooling module of a circulation and cooling method of liquid for refrigerant to cool the heat generation of the lamp unit 411, and the cooling module selectively adopts a direct water supply method or a non-direct water supply method for use.

Hereinafter, the bed-type near-infrared body care device 3000 of the present disclosure will be described.

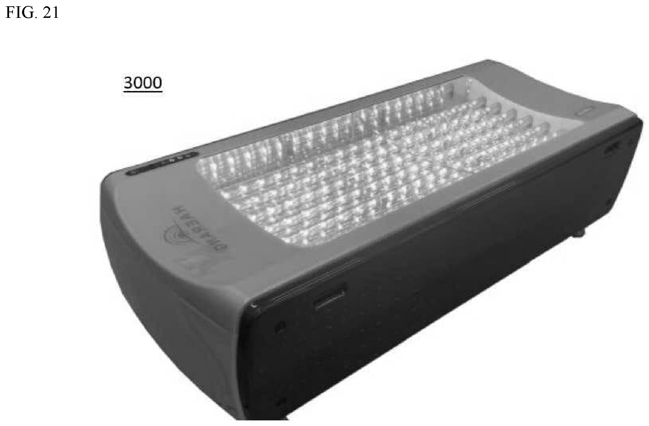

FIG. 21 is a product photograph of a bed-type near-infrared body care device according to yet another embodiment of the present disclosure.

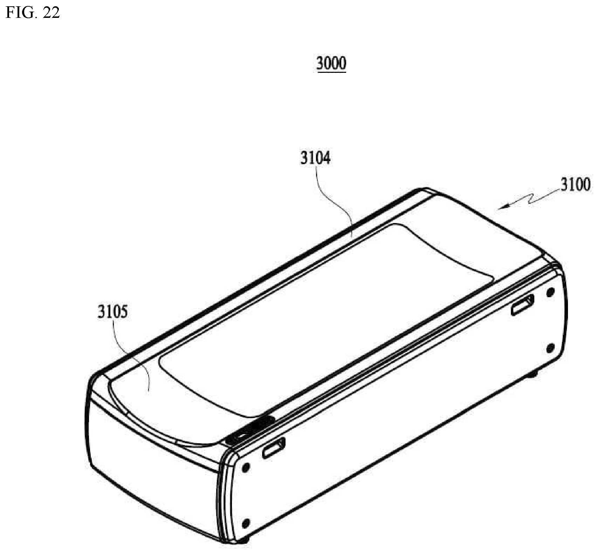

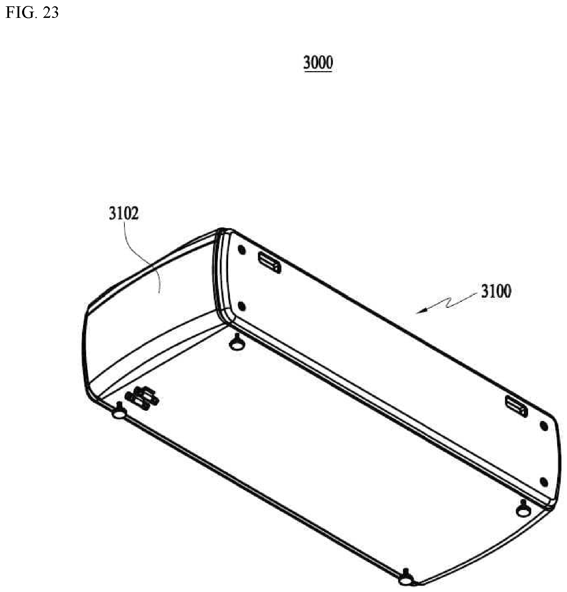

FIGS. 22 and 23 are perspective views of a bed-type near-infrared body care device according to yet another embodiment of the present disclosure.

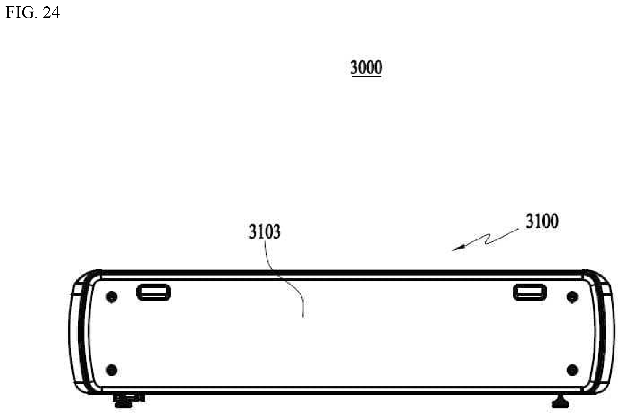

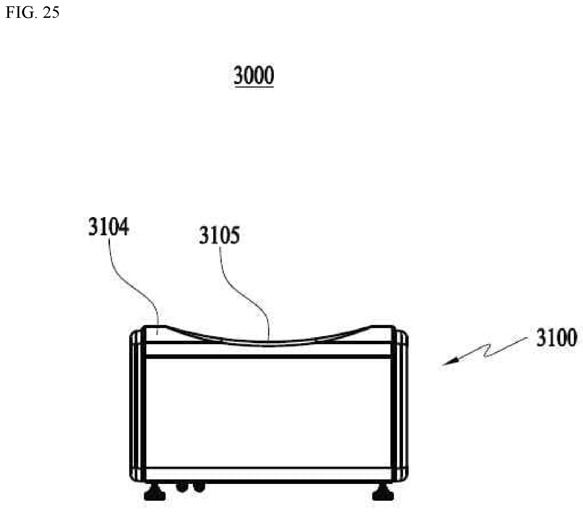

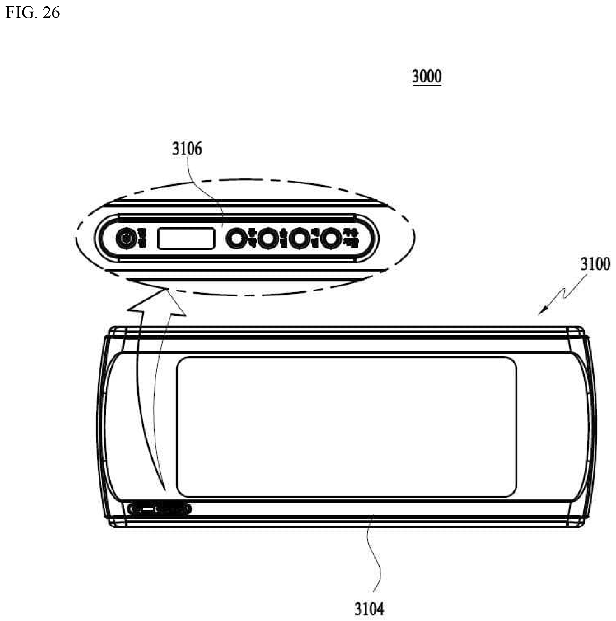

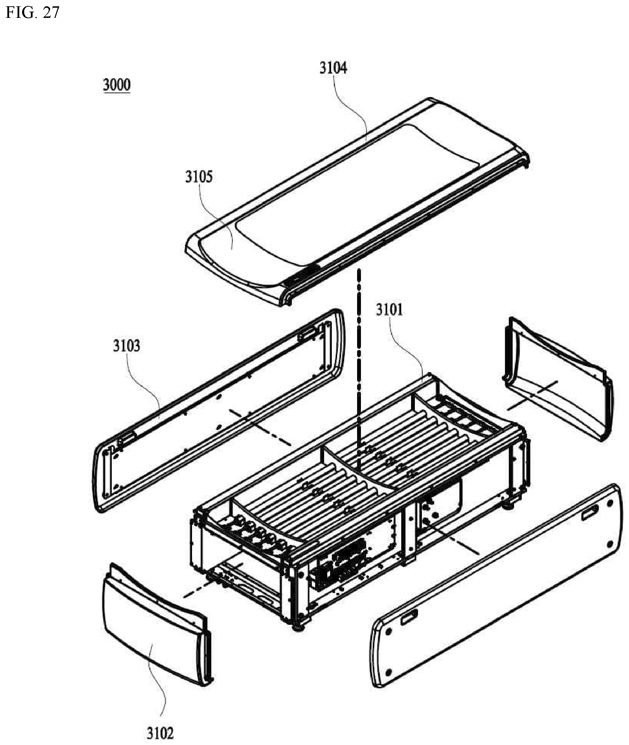

FIG. 24 is a side view of a bed-type near-infrared body care device according to yet another embodiment of the present disclosure, FIG. 25 is a front view of a bed-type near-infrared body care device according to yet another embodiment of the present disclosure, FIG. 26 is a plan view illustrating an operation control unit in a bed-type near-infrared body care device according to yet another embodiment of the present disclosure, and FIG. 27 is an exploded perspective view of a bed-type near-infrared body care device according to yet another embodiment of the present disclosure.

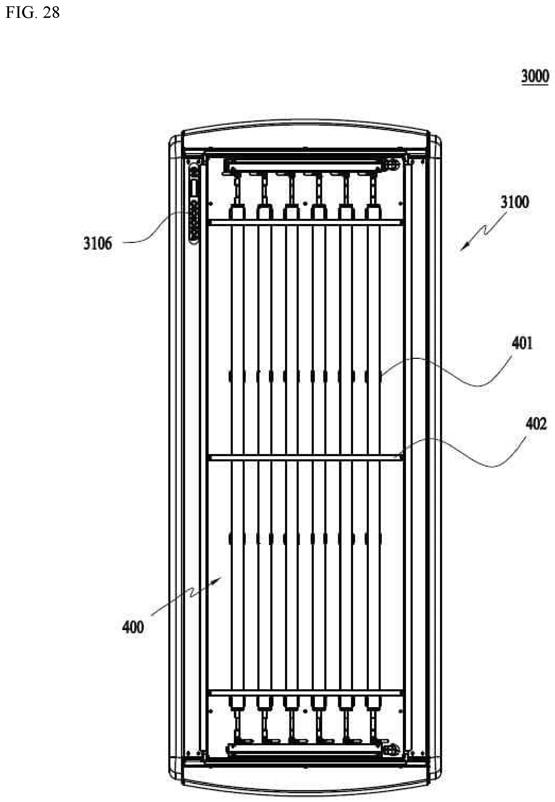

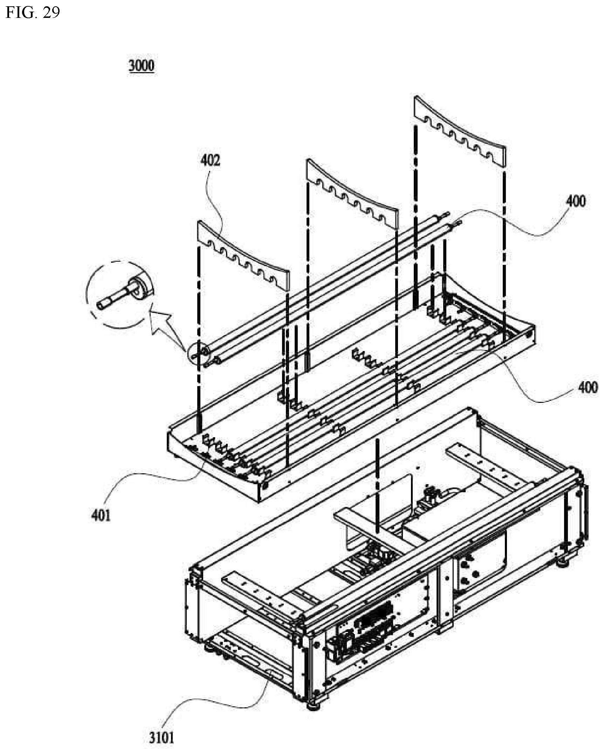

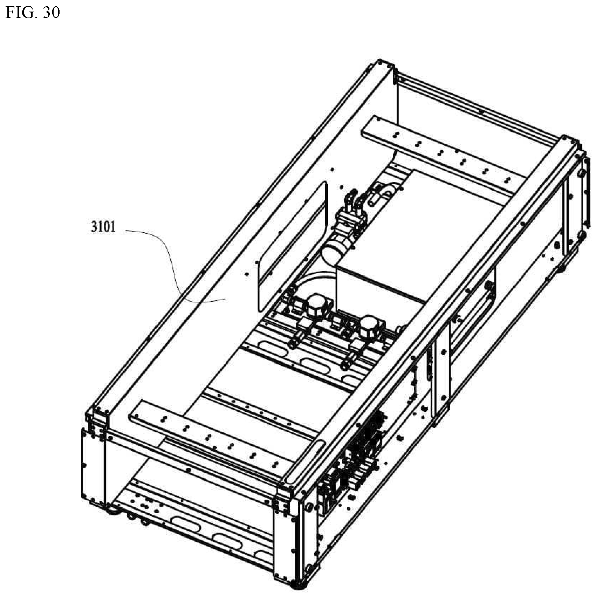

FIG. 28 is a plan view for explaining the installation structure of a lamp unit in a bed-type near-infrared body care device according to yet another embodiment of the present disclosure, and FIG. 29 is an exploded perspective view for explaining the installation structure of a lamp unit in a bed-type near-infrared body care device according to yet another embodiment of the present disclosure. And FIG. 30 is a perspective view illustrating a main frame in a bed-type near-infrared body care device according to yet another embodiment of the present disclosure.

Referring to FIGS. 21 to 30, the bed-type near-infrared body care device 3000 of the present disclosure comprises a bed body 3100 supported on the floor where the user can lie down; and a near-infrared lamp assembly 400 installed inside the bed body 3100 and configured so that far-infrared and mid-infrared rays among the emitted infrared rays are blocked and only near-infrared rays are emitted to penetrate deeply into subcutaneous tissue when the near-infrared rays are irradiated on the skin.

The base body 3100 includes a cooling module of a circulation and cooling method of liquid for refrigerant to cool the heat generation of the lamp unit 411, and the cooling module is configured to selectively adopt a direct water supply method or a non-direct water supply method for use.

In the base body 3100, front and rear frames 3102 are installed at the front and rear of the main frame 3101, left and right frames 3103 are installed on the left and right of the main frame 3101, and an upper frame 3104 is installed at the top.

A round-shaped groove 3105 is formed in the upper frame 3104 so that the user can lie down stably. An operation control panel 3106 capable of turning on/off and controlling near-infrared treatment is installed on one side of the upper frame 3104.

The near-infrared lamp assembly 400 of the present disclosure is fixed by a lower fixing bracket 401, and is configured to be aligned and positioned stably by an upper holder 402.

The internal double-tube structure of the near-infrared lamp assembly 400 of the present disclosure, as shown in FIG. 1, consists of a lamp unit 411 that emits infrared rays; and a double-tube lamp 410 accommodating the lamp unit 411 inside, formed with a central tube 412 and a light transmission tube 413 with a certain gap G between the inner and outer circumferences, and having double-tube caps 417 installed at both ends to allow liquid for refrigerant 414 to be charged and flow into the gap G between the central tube 412 and the light transmission tube 413.

The operational effects of the near-infrared body care system according to the preferred embodiment of the present disclosure configured as described above will be described as follows.

Sunlight is called radiation, and when arranged in order of long wavelengths, as shown in the figure, it consists of infrared rays, visible light, ultraviolet rays, X-rays, etc. The effect of such radiation on the human body is related to the length and shortness of the wavelength. According to a simple energy law, it has been expressed and used by Planck in the formula E=hv. h is Planck's constant, v is frequency, and λ is wavelength. Wavelength and frequency are in inverse proportion. With the formula v=1/λ, when the wavelength becomes longer, the frequency value becomes smaller. Conversely, when the wavelength becomes shorter, the frequency value becomes larger. Therefore, since radiation with a wavelength size greater than or equal to visible light has a correspondingly smaller energy value, it has less effect on the human body. Visible light is light that plays a role in making objects visible around us, and ultraviolet rays, X-rays or γ-rays have short wavelengths, so they emit a lot of energy and the energy penetrates deeply into the human body to cause harm. For example, ultraviolet rays have short wavelengths and cannot penetrate the skin, so they are mainly used for skin treatment, and the ultraviolet rays are light with a wavelength range between 200 and 400 nm. The wavelength of visible light (400-800 nm) is longer than the wavelength of ultraviolet rays and displays seven colors according to the wavelength so that we can distinguish colors. It can be said to be a wavelength that exists everywhere around us and is completely harmless.

Infrared rays are again divided according to wavelength into near-infrared rays in the 0.76-1.5 micrometer range, mid-infrared rays in the 1.5-5.6 micrometer range, and far-infrared rays in the 5.6-1,000 micrometer range. Among these, far-infrared rays in the 6-16 micrometer wavelength range are known to be most beneficial to our daily life. Infrared rays with wavelengths of 0.75-3 micrometers, which are outside the end of the red spectrum when light emitted from sunlight or a heating element is dispersed into a spectrum, are infrared rays, and among them, those with the shortest wavelength are near-infrared rays. Infrared rays have considerably longer wavelengths compared to ultraviolet rays, X-rays or γ-rays, so they act completely harmlessly on the human body. Therefore, in recent years, infrared rays generally have stronger thermal action compared to visible light or ultraviolet rays, so they are widely used for industrial or medical purposes.

Near-infrared rays exhibit photographic action, photoelectric action, and fluorescent action in addition to thermal action, so photographic plates, photocells, phototubes, thermocouples, phosphors, etc. are used as detectors, and they are also used for disinfection, sterilization, and treatment of joints and muscles.

In this way, all materials with heat emit infrared rays, and among them, solar radiation is the most important natural light source of infrared rays, and about 60% of the irradiation amount from the sun consists of infrared rays. Near-infrared rays have short wavelengths, and far-infrared rays have long wavelengths. Far-infrared rays penetrate about 2 mm into the skin, and near-infrared rays penetrate more than 10 mm up to 40 mm to activate our life cells. It can be said to be the light of life.

In the past, natural treatment was performed using sunlight and firelight, but for modern people, it has become difficult to benefit from these because it is not easy to be with nature in their busy lives. In particular, health is being lost in a polluted environment such as polluted air, water, consumption of various instant foods, stress, etc. Methods of treating diseases and maintaining a healthy life are also sought from nature, and one of them is near-infrared light, a natural light source of sunlight.

When light emitted from sunlight or a heating element is dispersed into a spectrum, infrared rays are outside the end of the red line, and among them, the electromagnetic wave with the shortest wavelength is near-infrared rays. Classified according to wavelength length, generally 0.75-3µm is called near-infrared (NIR:IRA), 3-25µm is called infrared (IRB), and more than 25µm is called far-infrared (FIR:IRC).

Infrared rays generally have thermal action compared to visible light or ultraviolet rays and are widely used for medical or industrial purposes, and are mainly used for disinfection, sterilization, and treatment of joints and muscles. In particular, near-infrared rays penetrate up to 6mm of the skin's subcutaneous layer and have excellent heat transfer, generating ATP and nitric oxide, greatly contributing to disease treatment.

Near-infrared rays are known to have no side effects and are simple to use. Rather, near-infrared rays can help health by obtaining skin beauty effects, massage effects, neuralgia prevention effects, and fatigue recovery effects just by being exposed to light for 10 minutes a day. In addition, NIR sunlight is mainly used for treating wounds, lacerations, scars, especially inflammation, and is also used for treating skeleton, joints, muscles, etc.

When absorbed in tissue, nitric oxide (NO) can be released from vascular endothelial cells and red blood cells, and tissue blood flow increases, and pain is reduced by oxidation and nitric oxide. In addition, it helps antibacterial agents in the tissue, and it is reported that the effect of helping wounds heal quickly is excellent.

In addition, near-infrared rays emitted from existing LEDs do not extract enough light to raise deep body temperature in a short time, and LEDs themselves do not generate heat but artificially create only near-infrared wavelengths using other components, so they are not used for active treatment and are applied for simple skin improvement or muscle care.

Other existing products using halogen lamps have the disadvantage that light cannot be directly irradiated on the eyes during near-infrared treatment because they can cause serious problems to the eyes, especially from blue light and light heat, but in the near-infrared body care system of the present disclosure, tungsten lamps are applied and have obtained the photobiological international safety standard IEC6271 that is safe especially for the eyes and human body from IEC (global safety certification organization).

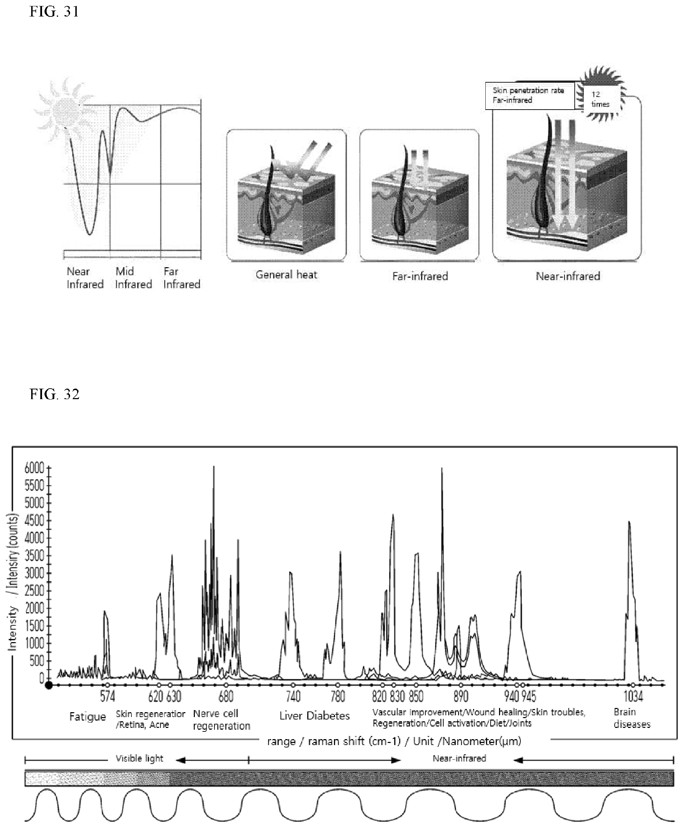

Meanwhile, FIGS. 31 and 32 are views explaining the penetration rate of deep penetration into subcutaneous tissue when near-infrared radiation is irradiated on the skin during near-infrared treatment in the near-infrared body care system according to the present disclosure and the therapeutic effects on various diseases.

In the near-infrared body care system according to the present disclosure, accommodating a lamp unit that emits infrared rays inside, formed with a central tube and a light transmission tube with a certain gap between the inner and outer circumferences, and providing a double-tube lamp with double-tube caps installed at both ends to allow liquid for refrigerant (for example, water) to be charged and flow into the gap between the central tube and the light transmission tube, and adopting a water filter method which is a filtration method using water as a medium, far-infrared and mid-infrared rays among the infrared rays emitted from the lamp unit are blocked and only near-infrared rays are emitted to the outside of the double-tube lamp, thereby enabling various health effects on the body by allowing deep penetration into subcutaneous tissue when the near-infrared rays are irradiated on the skin.

Referring to FIGS. 31 and 32, near-infrared rays have a skin penetration rate more than 12 times higher than far-infrared rays, so they can promote various health effects on the body by applying very high intensity heat to various diseases (for example, liver, diabetes, blood vessels, skin troubles, cell activation, brain diseases, etc.).

The near-infrared body care system of the present disclosure has the following effects.
① Combination treatment with high-frequency cancer treatment for various cancer and tumor patients
② Treatment related to immunity such as chronic skin diseases and various allergies
③ Treatment to improve blood circulation such as spinal cord/brain injury stroke, heart attack
④ Beauty care such as detox (waste discharge), skin and hair care, and diet
⑤ Immunity and thermal treatment necessary for treating various diseases due to aging
⑥ Treatment of male and female reproductive organ-related diseases (prostate, menstrual disorders, etc.)
⑦ Treatment related to muscle pain, inflammation, joints and peripheral nerves
⑧ Prevention and management of various diseases for mothers before and after childbirth (edema, depression, obesity, etc.)
⑨ Healthcare for prevention of various diseases due to lack of immunity

Meanwhile, the present disclosure may have the following characteristics.

The near-infrared lamp assembly 400 according to the present disclosure comprises a lamp unit 411 that emits infrared rays; and a double-tube lamp 410 accommodating the lamp unit 411 inside, formed with a central tube 412 and a light transmission tube 413 with a certain gap G between the inner and outer circumferences, and having double-tube caps 417 installed at both ends to allow liquid for refrigerant 414 to be charged and flow into the gap G between the central tube 412 and the light transmission tube 413, wherein the lamp unit 411 has a filament installed inside a glass body 411a, an inert gas (for example, nitrogen gas) is sealed inside the lamp unit 411, and has the technical feature configured so that far-infrared and mid-infrared rays among the infrared rays emitted from the lamp unit 411 are blocked and only near-infrared rays are emitted to the outside of the double-tube lamp 410, thereby enabling deep penetration into subcutaneous tissue when the near-infrared rays are irradiated on the skin.

The double-tube lamp 410 is configured to surround the outside of the lamp unit 411, and the liquid for refrigerant is charged and flows into the gap G, thereby cooling the high temperature heat emitted from the lamp unit 411 during near-infrared treatment and preventing the high temperature heat emitted from the lamp unit 411 from directly contacting the user's skin to prevent skin burns.

Meanwhile, the near-infrared body care system using the near-infrared lamp assembly according to the present disclosure has the technical feature of performing near-infrared treatment using the near-infrared lamp assembly 400 and comprising any one of a portable near-infrared body care device 1000, a chamber-type near-infrared body care device 2000, or a bed-type near-infrared body care device 3000.

First, the portable near-infrared body care device 1000 of the present disclosure comprises a support case 100 supported on the floor; a connection frame 200 having one end connected to the support case 100 and being extendable upward from the support case 100; and an action frame 300 connected to the other end of the connection frame 200 and provided with a near-infrared lamp assembly 400.

The near-infrared lamp assembly 400 comprises a lamp unit 411 that emits infrared rays; and a double-tube lamp 410 accommodating the lamp unit 411 inside, formed with a central tube 412 and a light transmission tube 413 with a certain gap G between the inner and outer circumferences, and having double-tube caps 417 installed at both ends to allow liquid for refrigerant 414 to be charged and flow into the gap G between the central tube 412 and the light transmission tube 413.

The lamp unit 411 has a filament installed inside a glass body 411a, an inert gas (for example, nitrogen gas) is sealed inside the lamp unit 411, and has the technical feature configured so that far-infrared and mid-infrared rays among the infrared rays emitted from the lamp unit 411 are blocked and only near-infrared rays are emitted to the outside of the double-tube lamp 410, thereby enabling deep penetration into subcutaneous tissue when the near-infrared rays are irradiated on the skin.

The double-tube lamp 410 is configured to surround the outside of the lamp unit 411, and the liquid for refrigerant is charged and flows into the gap G, thereby cooling the high temperature heat emitted from the lamp unit 411 and preventing the high temperature heat emitted from the lamp unit 411 from directly contacting the user's skin to effectively prevent skin burns.

The support case 100 includes a cooling module of a circulation and cooling method of liquid for refrigerant to cool the heat generation of the lamp unit 411, and the cooling module adopts a non-direct water supply method (refrigerant liquid self-circulation method) to facilitate movement and transportation of the near-infrared body care device.

The cooling module comprises a cooling unit 110 for receiving and cooling the liquid from the near-infrared lamp assembly 400; a liquid storage tank 120 for accommodating the liquid for refrigerant cooled by the cooling unit 110; and a pump 130 for receiving the liquid from the liquid storage tank 120 and pumping it to the near-infrared lamp assembly 400.

The cooling module uses a water filter to prevent contamination inside the liquid for refrigerant.

In the portable near-infrared body care device 1000 of the present disclosure, when viewed from a plan or side, the action frame 300 has a shape bent toward the opposite side with respect to the position of the connection frame 200, and a plurality of double-tube lamps 410 are installed on the side of the action frame 300 opposite to the position of the connection frame 200.

In the action frame 300, the lamp installation reflection bracket 310 bent along the bent shape is installed on the side opposite to the position of the connection frame 200, and the plurality of double-tube lamps 410 are installed side by side.

The lamp installation reflection bracket 310 has a container shape with an open lamp entry portion 311 facing the double-tube lamps 410 to accommodate the plurality of double-tube lamps 410, installation grooves are respectively formed in the opposite side flanges 312 of the lamp installation reflection bracket 310 into which first liquid flow tubes 440 extending from both ends of the double-tube lamps 410 are inserted, and elastic support pieces 415 supporting the vicinity of both ends of the double-tube lamps 410 are installed at the bottom of the lamp installation reflection bracket 310.

Furthermore, in the portable near-infrared body care device 1000 of the present disclosure, when viewed from the side, the action frame 300 is provided with distribution pipes 320 bent along the bent shape on the side where the double-tube lamps 410 are arranged, and when viewed from the front, the distribution pipes 320 are respectively installed on the left and right with the double-tube lamps 410 in between, a plurality of second liquid flow tubes 321 extending toward the first liquid flow tubes 440 are formed on the inner surface of each distribution pipe 320, and flexible tubes 330 are connected between the spaced first liquid flow tubes 440 and second liquid flow tubes 321.

A compressor 111 and a condenser 112 are installed at the bottom of the support case 100, a heat radiation fan 114 is installed on a side or lower surface of the support case 100, and the liquid storage tank 120 and a pump 130 for pumping the liquid for refrigerant are installed inside the support case 100.

The compressor 111 and the condenser 112 are sequentially arranged from front to rear while being surrounded by a protective frame 150 having a channel-shaped cross-section, the heat radiation fan 114 is arranged on the rear surface of the support case 100 to face the condenser 112, and the liquid storage tank 120 and the pump 130 are mounted on the top of the protective frame 150.

An evaporator 113 constituting the cooling unit 110 is installed inside the liquid storage tank 120.

The pump 130 sucks low-temperature liquid from the liquid storage tank 120 and supplies it to the double-tube lamps 410, and the high-temperature liquid discharged from the double-tube lamps 410 is resupplied to the liquid storage tank 120 and cooled by the evaporator 113.

The connection frame 200 connected to the support case 100 and the action frame 300 connected to the other end of the connection frame 200 are configured to be capable of horizontal rotation and elevation rotation.

A first connection pipe 610 and a second connection pipe 620, one end of which is connected to the distribution pipe 320, extend respectively on the left and right sides with respect to the connection frame 200 in the action frame 300, and the other ends of the first connection pipe 610 and the second connection pipe 620 are respectively connected to the support case 100 and connected to the liquid storage tank 120.

The connection frame 200 and the action frame 300 are detachably installed, and the first connection pipe 610 and the second connection pipe 620 are detachably installed to the action frame 300.

Furthermore, in the portable near-infrared body care device 1000 of the present disclosure, a biosensor unit S that detects user health information; a smart band or smart watch W that receives the user health information detected by the biosensor unit and that can be worn by the user to check health status; and a mobile communication terminal T configured with an installed application so that the health status improved by near-infrared treatment of the near-infrared body care device can be visually confirmed; are further included.

The biosensor unit S is installed inside the action frame 300 to photograph the user's body, for example, the head portion, and can precisely check the user's health status based on artificial intelligence.

Hereinafter, the chamber-type near-infrared body care device 2000 of the present disclosure will be described.

The chamber-type near-infrared body care device 2000 of the present disclosure comprises a base body 2100 supported on the floor and having a space portion where the user can lie down; a cover 2200 for opening and closing the base body 2100; and a near-infrared lamp assembly 400 installed inside the base body 2100 and configured so that far-infrared and mid-infrared rays among the emitted infrared rays are blocked and only near-infrared rays are emitted to penetrate deeply into subcutaneous tissue when the near-infrared rays are irradiated on the skin.

The base body 2100 includes a cooling module of a circulation and cooling method of liquid for refrigerant to cool the heat generation of the lamp unit 411, and the cooling module selectively adopts a direct water supply method or a non-direct water supply method for use.

Hereinafter, the bed-type near-infrared body care device 3000 of the present disclosure will be described.

The bed-type near-infrared body care device 3000 comprises a bed body 3100 supported on the floor where the user can lie down; and a near-infrared lamp assembly 400 installed inside the bed body 3100 and configured so that far-infrared and mid-infrared rays among the emitted infrared rays are blocked and only near-infrared rays are emitted to penetrate deeply into subcutaneous tissue when the near-infrared rays are irradiated on the skin.

The base body 3100 includes a cooling module of a circulation and cooling method of liquid for refrigerant to cool the heat generation of the lamp unit 411, and the cooling module is configured to selectively adopt a direct water supply method or a non-direct water supply method for use.

Although preferred embodiments of the present disclosure have been disclosed in the specification and drawings, although specific terms have been used, they are only used in a general sense to easily explain the technical content of the present disclosure and help understanding of the invention, and are not intended to limit the scope of the present disclosure.

It is obvious to those skilled in the art to which the present disclosure belongs that other modified examples based on the technical idea of the present disclosure can be implemented in addition to the embodiments disclosed herein.

### Reference numerals

- 1: Near-infrared body care system
- 100: Support case
- 110: Cooling unit
- 120: Liquid storage tank
- 130: Pump
- 170: Main controller
- 180: Check valve
- 190: Flow switch
- 200: Connection frame
- 300: Action frame
- 310: Lamp installation reflection bracket
- 311: Lamp entry portion
- 312: Flange
- 313: Installation groove
- 320: Distribution pipe
- 321: Second liquid flow tube
- 330: Flexible tube
- 400: Near-infrared lamp assembly
- 410: Double-tube lamp
- 411: Lamp unit
- 412: Central tube
- 413: Light transmission tube
- 414: Liquid
- 415: Elastic support piece
- 440: First liquid flow tube
- 610: First connection pipe
- 620: Second connection pipe
- 800: Liquid connection pipe
- 1000: Portable near-infrared body care device
- 2000: Chamber-type near-infrared body care device
- 3000: Bed-type near-infrared body care device

## Claims

1. A near-infrared body care device (1000) comprising:
a support case (100) supported on a floor;
a connection frame (200) having one end connected to the support case (100) and being extendable upward from the support case (100); and
an action frame (300) connected to the other end of the connection frame (200) and on which a near-infrared lamp assembly (400) is installed,
wherein the near-infrared lamp assembly (400) includes a plurality of double-tube lamps (410) arranged on the action frame (300),
each of the double-tube lamps (410) comprising:
a central tube (412) accommodating a lamp unit (411);
a light transmission tube (413) surrounding the central tube (412) in a spaced state; and
a liquid (414) flowing between the central tube (412) and the light transmission tube (413),
and the support case (100) comprising:
a cooling unit (110) for receiving and cooling the liquid (414) from the near-infrared lamp assembly (400);
a liquid storage tank (120) for accommodating the liquid cooled by the cooling unit (110); and
a pump (130) for receiving the liquid (414) from the liquid storage tank (120) and pumping it to the near-infrared lamp assembly (400).

2. The near-infrared body care device (1000) according to claim 1, wherein when viewed from a plan or side, the action frame (300) has a shape bent toward the opposite side with respect to the position of the connection frame (200), and a plurality of double-tube lamps (410) are installed on the side of the action frame (300) opposite to the position of the connection frame (200),
wherein a lamp installation reflection bracket (310) bent along the bent shape is installed on the side of the action frame opposite to the position of the connection frame (200), and the plurality of double-tube lamps (410) are installed side by side.

3. The near-infrared body care device (1000) according to claim 2, wherein the lamp installation reflection bracket (310) has a container shape with an open lamp entry portion (311) facing the double-tube lamps (410) to accommodate the plurality of double-tube lamps (410), installation grooves are respectively formed in the opposite side flanges (312) of the lamp installation reflection bracket (310) into which first liquid flow tubes (440) extending from both ends of the double-tube lamps (410) are inserted, and elastic support pieces (415) supporting the vicinity of both ends of the double-tube lamps (410) are installed at the bottom of the lamp installation reflection bracket (310).

4. The near-infrared body care device (1000) according to claim 3, wherein when viewed from the side, distribution pipes (320) bent along the bent shape are installed on the side of the action frame (300) where the double-tube lamps (410) are arranged, and when viewed from the front, the distribution pipes (320) are respectively installed on the left and right with the double-tube lamps (410) in between, and a plurality of second liquid flow tubes (321) extending toward the first liquid flow tubes (440) are formed on the inner surface of each distribution pipe (320), and flexible tubes (330) are connected between the spaced first liquid flow tubes (440) and second liquid flow tubes (321).

5. The near-infrared body care device (1000) according to claim 1, wherein a compressor (111) and a condenser (112) are installed at the bottom of the support case (100), a heat radiation fan (114) is installed on a side of the support case (100), and the liquid storage tank (120) and the pump (130) for pumping liquid are installed inside the support case (100).

6. The near-infrared body care device (1000) according to claim 5, wherein the compressor (111) and the condenser (112) are sequentially arranged from front to rear while being surrounded by a protective frame (150) having a channel-shaped cross-section, the heat radiation fan (114) is arranged on the rear surface of the support case (100) to face the condenser (112), and the liquid storage tank (120) and the pump (130) are mounted on the top of the protective frame (150).

7. The near-infrared body care device (1000) according to claim 5, wherein an evaporator (113) constituting the cooling unit (110) is installed inside the liquid storage tank (120).

8. The near-infrared body care device (1000) according to claim 7, wherein the pump (130) is configured to suck low-temperature liquid from the liquid storage tank (120) and to supply it to the double-tube lamps (410), and high-temperature liquid discharged from the double-tube lamps (410) is supplied to the liquid storage tank (120) and cooled by the evaporator (113).

9. The near-infrared body care device (1000) according to claim 5, wherein the connection frame (200) connected to the support case (100) and the action frame (300) connected to the other end of the connection frame (200) are configured to be capable of horizontal rotation and elevation rotation.

10. The near-infrared body care device (1000) according to claim 9, further comprising a first connection pipe (610) and a second connection pipe (620), one end of which is connected to the distribution pipe (320), the first connection pipe (610) and the second connection pipe (620) extending respectively on a left and right sides with respect to the connection frame (200) in the action frame (300), and the other ends of the first connection pipe (610) and the second connection pipe (620) being respectively connected to the support case (100) and connected to the liquid storage tank (120).

11. The near-infrared body care device (1000) according to claim 10, wherein the connection frame (200) and the action frame (300) are detachably installed, and the first connection pipe (610) and the second connection pipe (620) are detachably installed to the action frame (300).

12. The near-infrared body care device (1000) according to claim 5, further comprising:
a smart band or smart watch (W) that can be worn by a user to check health status; and
a mobile communication terminal (T) configured with an installed application so that the health status improved by the near-infrared body care device (1000) can be visually confirmed.

13. The near-infrared body care device (1000) according to claim 5, wherein a camera is installed on the action frame (300) toward the user's head.
